(19) Europäisches Patentamt / European Patent Office / Office européen des brevets

(11) EP 3 750 882 A1

(12) **EUROPEAN PATENT APPLICATION**
published in accordance with Art. 153(4) EPC

(43) Date of publication:
**16.12.2020 Bulletin 2020/51**

(21) Application number: **19768507.6**

(22) Date of filing: **08.03.2019**

(51) Int Cl.:
***C07D 307/32*** *(2006.01)*
***A61K 31/365*** *(2006.01)*
***A23L 33/10*** *(2016.01)*
***A61P 31/00*** *(2006.01)*
***A61P 37/00*** *(2006.01)*

(86) International application number:
**PCT/KR2019/002757**

(87) International publication number:
**WO 2019/177314 (19.09.2019 Gazette 2019/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2018 KR 20180028871**

(71) Applicant: **Enzychem Lifesciences Corporation**
**Chungcheongbuk-do 27159 (KR)**

(72) Inventors:
- **SOHN, Ki Young**
  **Seoul 07264 (KR)**
- **KIM, Jae Wha**
  **Daejeon 34140 (KR)**
- **YOON, Sun Young**
  **Daejeon 34199 (KR)**
- **YOO, Chang Hyun**
  **Daejeon 34010 (KR)**
- **JEONG, Jin Seon**
  **Cheonan-si, Chungcheongnam-do 31139 (KR)**

(74) Representative: **Henkel & Partner mbB**
**Patentanwaltskanzlei, Rechtsanwaltskanzlei**
**Maximiliansplatz 21**
**80333 München (DE)**

(54) **DIACYLGLYCEROL LACTONE COMPOUND, PREPARATION METHOD THEREFOR, AND IMMUNOSTIMULATOR CONTAINING SAME AS ACTIVE INGREDIENT**

(57) Disclosed are a novel diacylglycerol lactone compound that promotes neutrophil migration to enhance immunity and inhibit infection, a method for preparing the same, and an immunostimulator containing the same as an active ingredient. The diacylglycerol lactone compound is represented by Chemical formula 1 in the specification. In Chemical formula 1, R1 and R2 are independently a fatty acid residue of 2 to 30 carbon atoms.

Fig. 1

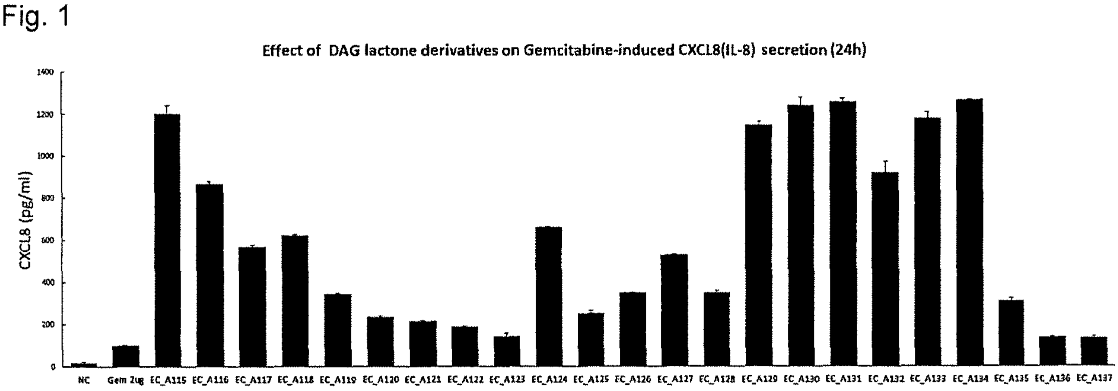


EP 3 750 882 A1

## Description

### Technical Field

The present invention relates to a diacylglycerol lactone compound, and more particularly, to a novel diacylglycerol lactone compound for promoting neutrophil migration to enhance immunity and to suppress infection, a method for preparing the same and an immunostimulator comprising the same as active ingredient.

### Background Art

Immunity is a self-defense response that physiologically recognizes, eliminates and/or metabolizes exogenous and endogenous foreign substances in a body. The immune response can be classified into an innate immunity which is an initial immune response and an acquired immunity which is a late immune response. In the initial immune response, a host is protected by suppressing foreign substances (pathogens) by the activities of macrophages and natural killer cells (NK cells). At this time, the macrophages phagocytize foreign substances and produce and secrete TNF-$\alpha$ which is an active marker. NK cells destruct pathogen-infected cells by producing and secreting perforin which is an active marker. Subsequently, cytotoxic T lymphocytes, helper T lymphocytes, and B lymphocytes, which are involved in the acquired immunity, are activated to kill infected cells or produce antibodies thereby to protect the host. The cytotoxic T lymphocytes kill pathogen-infected cells by producing and secreting a lot of perforin like NK cells. B lymphocytes protect the host by producing antibodies, either dependent or independent of helper T lymphocytes. Inflammatory cytokines such as IL-6, IL-8, TNF-$\alpha$ and so on are substances that mediate the immune response and are known to be particularly involved in the initial immune response.

Generally, in case of immunodeficiency, resistance to infection is lowered, patients with inadequate antibody production cannot protect themselves against bacterial infection, and phagocytosis ability of neutrophils is also lowered. Further, in this case, since an activation of a complement system is also suppressed, leukocyte migration factors, etc. are not produced, so that the inflammation increases and viralemia occurs, causing the virus to spread to the central nervous system or elsewhere. In case of cancer patients, during chemotherapy or radiation treatment, not only cancer cells but also normal cells are affected, and the patient's immunity may rapidly decrease as a side effect.

Immunostimulators are drugs for treating congenital and acquired immunodeficiency, and immunoglobulin, interferon (INF), and the like are representatively used as the immunostimulators. Among these, a preparation obtained by concentrating and purifying IgG, which is one of human immunoglobulins, is used for the prevention and treatment of measles, chickenpox, hepatitis B, mumps, etc. However, the preparation has disadvantages of pain at the injection site and lowering blood pressure. Interferon was discovered as an antiviral factor, but afterwards, its cell proliferation inhibitory action, its immune modulating action, etc. were discovered, and it is used as an antiviral agent and an antitumor agent. However, the type $\beta$ thereof has side effects such as fever, boredom, loss of appetite, local pain for injection, and alopecia, while the type $\alpha$ thereof may cause side effects such as reduction of white blood cells due to temporary suppression of bone marrow function.

### Disclosure of Invention

An object of the present invention is to provide a novel diacylglycerol lactone compound having an immunity enhancing effect and a method for preparing the same.

Another object of the present invention is to provide a diacylglycerol lactone compound for enhancing immunity and inhibiting infection by increasing the expression of IL-8 (CXCL8) and promoting neutrophil migration, and an immunostimulator containing the diacylglycerol lactone compound as an active ingredient.

In some embodiments for achieving the above objects, the present invention provides a diacylglycerol lactone compound represented by following formula 1.

[Chemical formula 1]

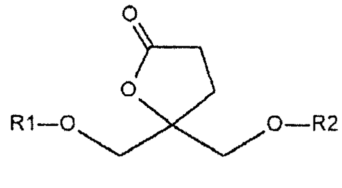

In Chemical formula 1, R1 and R2 each is independently a fatty acid residue of 2 to 30 carbon atoms.

In addition, the present invention provides an immunostimulator and a health functional food composition for immunity enhancement comprising the diacylglycerol lactone compound represented by Chemical formula 1 as an active ingredient. In addition, the present invention provides a method for enhancing immunity comprising administering an immunostimulator containing a diacylglycerol lactone compound represented by Chemical formula 1 as an active ingredient to a non-human subject.

The diacylglycerol lactone compound of the present invention is a novel compound having an immunity enhancing effect. It increases the expression of IL-8 (CXCL8) and promotes neutrophil migration, thereby enhancing immunity and inhibiting infection.

## Brief Description of Drawings

Figure 1 is a graph showing an increase in the expression of CXCL8 (IL-8) in cells due to a diacylglycerol lactone compound according to the present invention.

Figure 2 is a graph showing a bacterial infection inhibitory effect of the diacylglycerol lactone compound according to the present invention.

Figure 3 is a photograph showing the results of the bacterial infection inhibition experiment of diacylglycerol lactone compound according to the present invention.

## Best modes for carrying out the Invention

Hereinafter, with reference to the accompanying drawings, the present invention will be described in detail.

The present invention provides a novel diacylglycerol lactone compound represented by the following Chemical formula 1.

[Chemical formula 1]

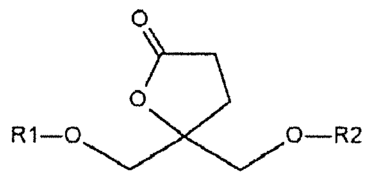

In Chemical formula 1, R1 and R2 each is independently a fatty acid residue of 2 to 30 carbon atoms, preferably 2 to 20 carbon atoms. Preferably R1 is a carbonyl group of 2 to 6 carbon atoms, preferably 2 to 3 carbon atoms (i.e., acetyl group or propionyl group), R2 is a fatty acid residue of 4 to 30 carbon atoms, preferably 4 to 20 carbon atoms. Specifically, R1 and R2 each is independently acetyl, propionyl, butyryl, isobutyryl, valeroyl, pivaloyl, 2-methylbutyryl, cyclopropanecarbonyl, cyclohexanecarbonyl, hexanoyl, heptanoyl, nonanoyl, dodecanoyl, myristoyl, palmitoyl, linoleoyl, oleoyl, linolenoyl, eicosanoyl, arachidonoyl and so on, preferably, acetyl, propionyl, butyryl, isobutyryl, cyclopropanecarbonyl, 2-methylbutyryl, pivaloyl, palmitoyl, linoleoyl and so on. The compounds of Chemical formula 1 is a racemic material or an optically active material.

Preferred examples of the diacylglycerol lactone compound represented by Chemical formula 1 may include a compound in which R1 or R2 is an acetyl group, and specific examples includes compounds represented by the following Chemical formulas 2a and 2b.

[Chemical formula 2a]

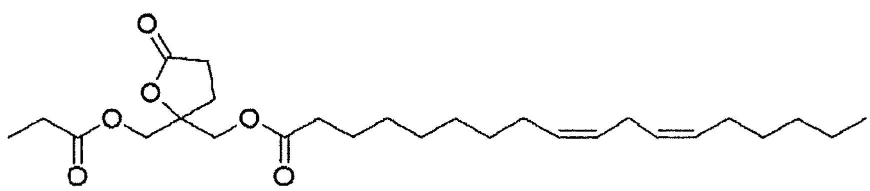

[Chemical formula 2b]

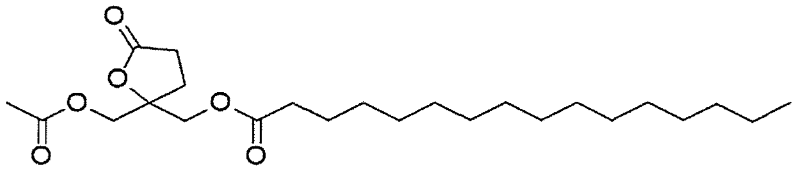

The diacylglycerol lactone compound represented by Chemical formula 1 can be prepared by the following Reactions 1 to 4.

[Reaction 1]

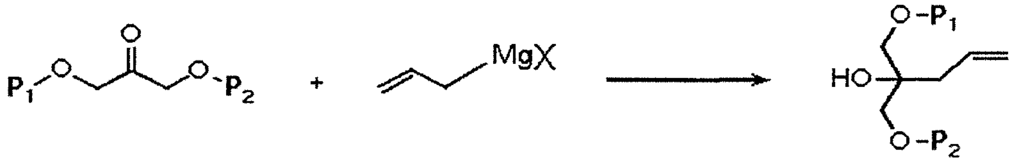

[A] [B]

Reaction 1 is a reaction for preparing a compound represented by Chemical formula B by reacting the compound represented by Chemical formula A with $CH_2$=CH-$CH_2$-MgX (X is halogen atom), wherein P1 and P2 are protecting groups. The protecting groups may be selectively removed during a deprotection reaction, and each independently can be 4-metoxyphenyl, benzyl, tert-butyldiphenylsilane and so on. As the solvent for the reaction, an organic solvent may be used, and preferably tetrahydrofuran, diethyl ether, dioxane, etc., which are ethereal solvents, may be used. The equivalent of $CH_2$=CH-$CH_2$-MgX (X is a halogen atom, for example, Br or Cl, specifically Cl) Grignard reagent is preferably 2 to 4 equivalents with respect to the reactant [A].

[Reaction 2]

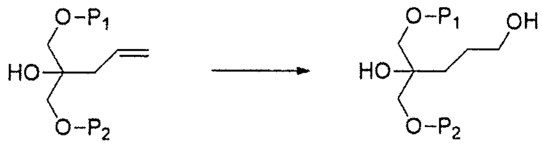

[B] [C]

Reaction 2 is a reaction for producing a compound represented by Chemical formula C by carrying out hydroxylation reaction of the double bond of Chemical formula B compound. As the reaction solvent, tetrahydrofuran may be used 15 to 30 times, preferably 20 times by volume with respect to the weight of compound [B]. In the Reaction 2, boron reacts with the double bond (boration) and then hydroxylation reaction proceeds. The amount of borane dimethylsulfide (BMS) used for the formation of a ring ether is 1.5 to 2 equivalents, preferably 1.6 to 1.7 equivalents.

[Reaction 3]

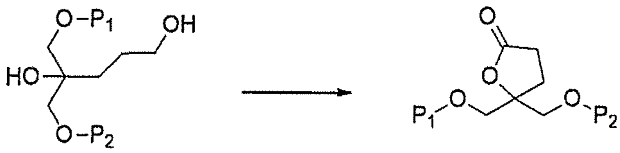

[C] [D]

Reaction 3 is a reaction to obtain a compound represented by Chemical formula D by cyclizing a compound of Chemical formula C by lactone cyclization reaction. As the reaction solvent, methylene chloride may be used 3 to 10

times, preferably 5 times by volume with respect to the weight of compound [B]. The amount of pyridinium chlorochromate ($C_5H_6NClCrO_3$, PCC) used for lactone formation is 9 to 11 equivalents, preferably 10 equivalents of compound [B].

[Reaction 4]

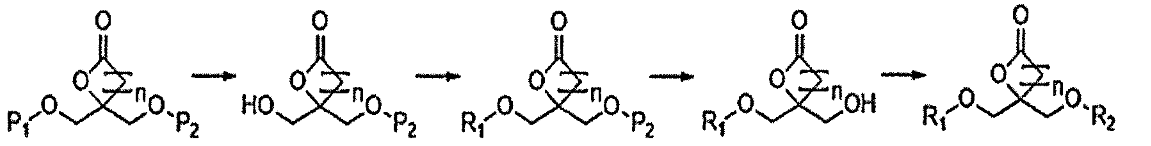

[D] [E] [F] [G] [CHEMICAL FORMULA 1]

Reaction 4 is a reaction to obtain a compound represented by Chemical formula 1 by deprotecting and esterifying the compound of Chemical formula D (deprotection and esterification reaction). Selective deprotection reaction of one side chain and an esterification reaction are sequentially carried out. R1 and R2 are as defined in Chemical formula 1. Selective deprotection agents include Ceric Ammonium Nitrate (CAN), Boron trichloride(1M in MC, $BCl_3$), tetrabutyl ammoniumfluoride(TBAF) and so on, and the amount thereof is 2 to 4 equivalents, preferably 3 equivalents of compound [D], [F]. As a solvent, a mixed solvent composed of acetonitrile and purified water and a methylene chloride can be used, and the amount thereof is 15 to 30 times, preferably 24 times by volume with respect to the weight of Compound [D].

In the esterification reaction of Reaction 4, a fatty acid (R1OH or R2OH) having a carbon atom number of 2 to 30 can react with pivaloyl chloride in a non-polar organic solvent in the presence of an organic base, to prepare a mixed anhydride(

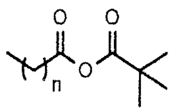

,

n= an integer of 0 to 20) of an activated form. Next, Compounds [E] and [G] and the mixed anhydride are reacted in the presence of 4-4-dimethylaminopyridine (4-dimethylamino pyridine, DMAP) to prepare the diacylglycerol lactone compound of Chemical formula 1.

The diacylglycerol lactone compound represented by Chemical formula 1 increases the expression of IL-8 (CXCL8) cytokine and promotes neutrophil migration from blood vessels to tissues, thereby enhancing immunity and inhibiting infection. Therefore, the diacylglycerol lactone compound of the present invention can be used to prevent or treat immune-related diseases. Examples of immune-related diseases that can be prevented or treated by the administration of the diacylglycerol lactone compound of the present invention may include various bacterial and viral infection diseases, acute or chronic inflammatory lung diseases, pneumonia, sepsis, and so on. As used herein, the term "prevention" or "preventing" includes any activity to suppress the decline of immunity or enhance immunity by administering the composition of the present invention. The term "treatment" or "treating" includes any activity to improve or beneficially alter the symptoms of immune-related diseases by the composition of the present invention.

The diacylglycerol lactone compound of the present invention increases the expression of CXCL8 (IL-8) in cells, promotes neutrophil migration, and inhibits bacterial infection bronchial fungal infection of animal models. Neutrophil, which is generally produced up to $10^{11}$ per day in normal people, matures from the bone marrow, then circulates in blood vessels for about 8 hours, penetrates into the tissues, survives for several days, and dies or disappears. When a bacterial infection occurs, the immune cells that primarily remove bacteria are neutrophils. Vascular endothelial cells are activated by chemokines (CXCL8 or CXCL2, etc.) and various inflammatory factors secreted from damaged tissues in the area infected with bacteria, thereby circulating neutrophil cells in the blood vessels to move to the tissue and to removes bacteria present in infected tissue. In the experimental example of the present invention, it was confirmed that the diacylglycerol lactone compound of Chemical formula 1 increased the expression of CXCL8 chemokine (IL-8) in THP-1 cells, which are cells of the human macrophage family (Experimental Example 2-1), and the diacylglycerol lactone compound of Chemical formula 1 inhibited bacterial infection in a bacterial fungal lung infection mouse model (Experimental Example 4-1).

Diacylglycerol lactone compound of present invention may be used as an immunostimulator alone without mixing other substance, or in the form of a pharmaceutical composition containing the diacylglycerol lactone compound as an active ingredient. When diacylglycerol lactone compound of present invention is used in the pharmaceutical composition, conventional pharmaceutically acceptable carriers, excipients, or diluents can be included therein. The amount of diacylglycerol lactone compound in the pharmaceutical composition can be widely varied without specific

limitation, and is specifically 0.0001 to 100.0 weight%, preferably, 0.001 to 95.0 weight%, more preferably 0.01 to 50 weight%, for example 1 to 20 weight%, with respect to the total amount of the composition.

The pharmaceutical composition may be formulated into any one selected from the group consisting of tablets, bolus, powders, granules, capsules, suspensions, liquid solutions, emulsions, syrups, sterilized aqueous solutions, non-aqueous solutions, suspensions, emulsions, freeze-dried agents, and suppositories and so on, and may be formulated into various forms for oral or non-oral administration. In formulating the composition, conventional excipients or diluents such as fillers, bulking agents, binders, wetting agents, disintegrating agents, and surfactants can be used. The solid formulation for oral administration includes tablet, bolus, powder, granule, capsule and so on, and such solid formulations can be prepared by mixing one or more of the components and at least one excipient such as starch, calcium carbonate, sucrose, lactose, gelatin, and so on. Besides the excipient, a lubricant such as Magnesium stearate and talc can also be used. The liquid formulation for oral administration includes suspension, liquid solutions, emulsion, syrup, and so on, and may include conventional diluents such as water and liquid paraffin or may include various excipients such as wetting agents, sweeting agents, flavoring agents, and preserving agents. The formulation for non-oral administration includes sterilized aqueous solution, non-aqueous solution, suspension, emulsion, freeze-dried formulation, suppository, and so on, and solvent for solution such as non-aqueous solution, suspension may include propylene glycol, polyethylene glycol, vegetable oils such as olive oil, and ester for syringe injection such as ethyl oleate. Base materials of the suppository may include witepsol, macrogol, tween 61, cacao butter, Laurin and glycerogelatin.

The composition of present invention can be administered in a pharmaceutically effective amount. The term "pharmaceutically effective amount" is used to refer to an amount that is sufficient to treat a disease at a reasonable benefit/risk ratio applicable to achieve a desired result in a medical treatment. The "pharmaceutically effective amount" can be determined according to the subject’s category, age, sex, severity and type of disease, activity of drug, sensitivity to drug, administration time, administration route, excretion rate, duration of treatment, factors including concurrent drugs, and other factors well known in the medical field. The composition of the present invention can be administered alone or with other therapeutic agents sequentially or simultaneously. The composition of the present invention can be administered once or multiple times. It is important to administer an amount capable of obtaining the maximum effect in a minimum amount without side effects in consideration of all of the above factors, which can be easily determined by a person skilled in the art. The preferable amount of the composition of the present invention can be varied according to the condition and weight of patient, severity of disease, formulation type of drug, administration route and period of treatment. An appropriate total amount of administration per 1 day can be determined by a physician, and is generally about 0.001 to about 1000 mg/kg, preferably about 0.05 to 200 mg/kg, more preferable about 0.1 to about 100 mg/kg once a day or can be administered in divided doses multiple times daily. The compound or composition can be applied to any subject without specific limitation as long as it is an individual for the purpose of preventing immunity reduction, of enhancing immunity, or of treating an immune disease. For example, the composition of the present invention can be administered to not only human but also non-human animal (specifically mammals) such as monkey, dog, cat, rabbit, guinea pig, rat, mouse, cow, sheep, pig, goat, and birds and fishes, and so on. The composition of the present invention can be administered by conventional various methods, for example, by oral or rectum administration, or by intravenous (i.v.), intramuscular (i.m.), subcutaneous (s.c.), intrauterine dural or cerebrovascular injection.

In some embodiments, the present invention provides health functional food compositions for enhancing immunity, which comprises a diacylglycerol lactone compound of formula 1 as an active ingredient. Specifically, the diacylglycerol lactone compound of the present invention may be included in a health functional food composition for preventing immunity lowering, enhancing immunity, preventing or improving immune-related diseases. The term "improvement" or "improving" refers to any activity to improve or ameliorate the symptoms of an individual who is suspicious of an immune-related disease or developing an immune-related disease.

The health functional food composition may consist of only or substantially pure compound of the present invention or may include compound of the present invention together with other conventional ingredients of health functional food. The amount of the active ingredient in the health food composition can be determined suitably according to the intended use. Generally, when the compound of the present invention is included in food or beverages, the amount of the composition according to the present invention is preferably less than 15 weight%, more preferably less than 10 weight %, with respect to the total amount of the raw material. In case of a long term use for the purpose of the health control and hygiene, the amount can be less than the above range. Since there is no problem in terms of safety, amount of the active component is greater than the above range.

Foods to which the compound of the present invention can be added are not limited, and include various foods, for example, meats, sausages, breads, chocolates, candies, snacks, pizzas, noodles, gums, daily products such as ice creams, soups, beverages, teas, drinks, alcoholic beverages, vitamin complexes and any health functional food, and also include food used as feed for animals. When the health functional food composition of present invention is used in the beverage product, the beverage product may include sweeting agents, flavoring agents or natural carbohydrates. Examples of natural carbohydrates include monosaccharides such as glucose and fructose, disaccharides such as maltose and sucrose, polysaccharides such as dextrin and cyclodextrin, and sugar alcohols such as xylitol, sorbitol and

erythritol. The amount of carbohydrate in the beverage composition can be widely varied without specific limitation, and is preferably 0.01 to 0.04 g, more preferably, 0.02 to 0.03 g per 100 ml of the beverage. Examples of sweeting agents include natural sweeteners such as thaumatin and stevia extract and artificial sweeteners such as saccharin and aspartame. In addition to the above, the health functional food composition of the present invention may include various nutrients, vitamins, electrolytes, flavoring agents, colorants, pectic acid and salts thereof, alginic acid and salts thereof, organic acids, protective colloidal thickening agents, pH controlling agents, stabilizing agents, preserving agents, glycerin, alcohol, carbonizing agents used in carbonated beverages and so on. Moreover, the health functional food composition of the present invention may include fruits, as used in preparing natural fruit juices and fruit juice beverages and vegetable beverages.

In some embodiments, the present disclosure provides methods for enhancing immunity or preventing or treating an immune-related disease, comprising administering the pharmaceutical composition to a patient in need thereof. The term "a patient in need" includes any animal including human that suffers from immune-related disease or can develop immune-related disease. Immune-related disease can be treated or prevented by administering an effective amount of a pharmaceutical composition containing a compound of the present invention or containing the compound of the present invention and pharmaceutically acceptable salt thereof to a patient in need thereof. The term "administration" means introducing the pharmaceutical composition of the present invention to a patient in need by any suitable method. The composition of the present disclosure can be administered by conventional various methods, for example, by oral or non-oral administration as far as the target organization can be reached. In some embodiments, the method of the present disclosure comprises administering a therapeutically effective amount of a pharmaceutical composition comprising diacylglycerol lactone compound of formula I to a patient in need thereof. An appropriate total amount of administration per 1 day can be determined by a physician, and is generally about 0.001 to about 1000 mg/kg, preferably, about 0.05 to 200 mg/kg, more preferably about 0.1 to about 100 mg/kg. The total administration amount per day can be administered once a day or can be administered in divided doses multiple times daily. However, the specific therapeutically effective amount of pharmaceutical composition administered to a particular patient can be varied depending on the type and degree of the response to be achieved in the treatment, the specific composition, including whether another agent is included in the composition, the patient's age, body weight, general health status, sex, diet, administration time, administration route, the ratio of composition, treatment period, other drugs used together in the treatment and a variety of factors well known in the medical field.

## Embodiments for carrying out the Invention

Hereinafter, the present invention is described in more detail through examples. The following example is only to help the understanding of the present invention, and the present invention is not limited by the following examples.

[Example 1] Synthesis of diacylglycerol lactone compound (EC-A129)

A. As shown in Reaction 1a below, 50g (402.77 mmole) of 4-methoxyphenol was dissolved in 1500 ml of acetone. Then, 278g (2013.8mmole) of $K_2CO_3$ was added and stirred at room temperature for 30 minutes. 126ml (1611.1mmole) of epichlorohydrin was added to the 4-methoxyphenol solution, the temperature was raised to 60 to 65 °C, and refluxed for 72 hours. The reaction was confirmed by TLC (ethylacetate(EA): hexane(Hex)=1:9). When the reaction was completed, the reaction solution was filtered by a celite filter, the filtrate was concentrated. The concentrate was purified with a flash column(eluent: ethyl acetate(EA): hexane(Hex) = 1:10 (volume ratio) mixture) to obtain 68g of the target compound(yield: 97.3%).

[Reaction 1a]

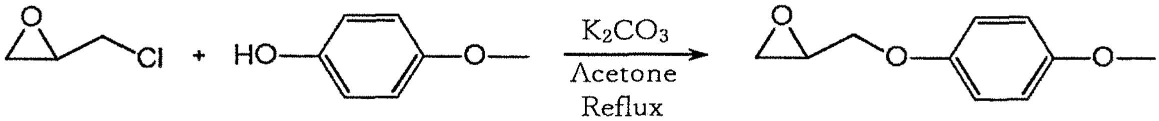


B. As shown in Reaction 1b below, 0.73ml (7.0755mmole) of benzylalcohol was dissolved in 4 ml of dimethylformamide (DMF). 283 mg (7.0755 mmole) of 60%-NaH was slowly added and stirred at room temperature for 30 minutes. 850 mg (4.717 mmol) of the product of Reaction 1a(SM) was dissolved in 3ml of dimethylformamide (DMF). The dissolved solution was slowly added dropwise to the reaction solution, followed by stirring at 80 °C for 3 hours.

The reaction was confirmed by TLC(EA:Hex=1:2). When the reaction was completed, $H_2O$ was added to the reaction solution to quench the reaction, extracted with ethyl acetate (EA) / H2O. Thereafter the organic layer was washed with purified water 3 times, water was removed with $MgSO_4$, and then concentrated. The concentrate was purified with a flash column (eluent: ethyl acetate (EA): hexane (Hex) = 1:4 (volume ratio) mixture) to obtain 1.07g of the target compound (yield: 78.3%).

[Reaction 1b]

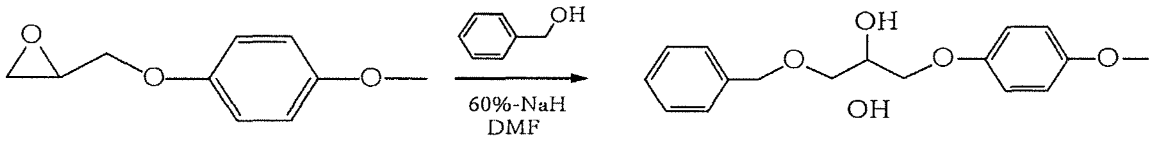


SM

C. As shown in Reaction 1c below, 52.77g (297.79 mmole) of pyridinium chlorochromate (PCC) and 52.77g of celite were added in 233.5 ml of methylene chloride (MC) and stirred. 23.55g (81.67mmole) of the product (SM) of Reaction 1b was dissolved in 81.2 ml of methylene chloride (MC) and it was slowly added dropwise. It was stirred at room temperature for 24 hours. The reaction was confirmed by TLC (EA:Hex=1:2). When the reaction was completed, it was filtered through a celite filter, and the filtrate was concentrated. The concentrate was purified with a flash column(eluent: ethyl acetate(EA): methylene chloride(MC):hexane(Hex) = 1:1:4(volume ratio) mixture) to obtain 14.2g of the target compound(yield: 60.7%).

[Reaction 1c]

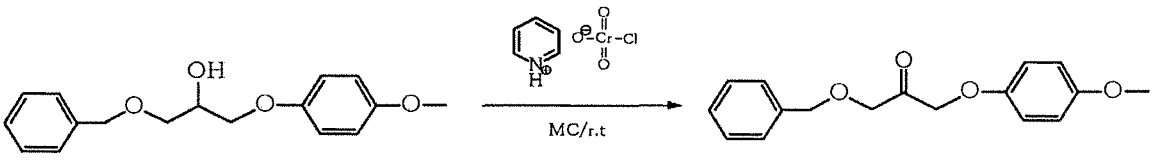


SM

D. As shown in Reaction 1d below, 370 mg (1.29 mmole) of the product(SM) of Reaction 1c was dissolved in 1.3 ml of tetrahydrofuran(THF). Then, after bubbling with nitrogen gas(N2), it was cooled to 0 °C. 1.94ml(3.877mmle) of allylmagnesium chloride (2M in THF) was slowly added dropwise and stirred at room temperature for 2 hours. The reaction was confirmed by TLC(EA: Hex=1:4). When the reaction was completed, a dilute aqueous hydrochloric acid solution was added to the reaction solution to quench the reaction. Then, it was extracted with ethyl acetate(EA)/H2O and then concentrated after removing moisture with magnesium sulfate ($MgSO_4$). The concentrate was purified with a flash column(eluent: ethyl acetate(EA): hexane(Hex) = 1:7(volume ratio) mixture) to obtain 250mg of the target compound(yield: 59%).

[Reaction 1d]

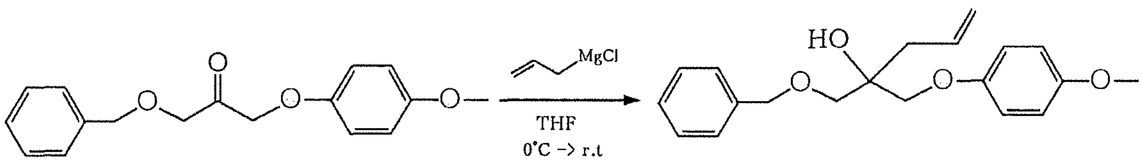


SM

E. As shown in Reaction 1e below, 6.5g (19.793 mmol) of the product(SM) of Reaction 1d was dissolved in 130 ml of tetrahydrofuran (THF). Then, after bubbling with nitrogen gas($N_2$), it was cooled to -78 °C. 16ml (31.977mmole) of borane dimethyl sulfide solution (2M in THF, $BH_3Me_2S$) was slowly added dropwise and stirred at the same temperature for overnight. The reaction was confirmed by TLC (EA:Hex=1:2). When the reaction was completed, methanol(MeOH) was added to the reaction solution to quench the reaction, followed by concentration. 42.6g(197.93mmole) of pyridine chlorochromate(PCC) and 42.6g of celite were added in 32.5ml of methylene

chloride(MC) and stirred. A solution in which the concentrated reactant SM2 was dissolved in an appropriate amount of methylene chloride(MC) was added and stirred at room temperature for 5 hours. The reaction was confirmed by TLC(EA:Hex=1:2). When the reaction was completed, it was filtered through a celite filter, and the filtrate was concentrated. The concentrate was purified with a flash column(eluent: ethyl acetate(EA): hexane(Hex) = 1:4(volume ratio) mixture) to obtain 4.56g of the target compound(yield: 67.3%).

[Reaction 1e]

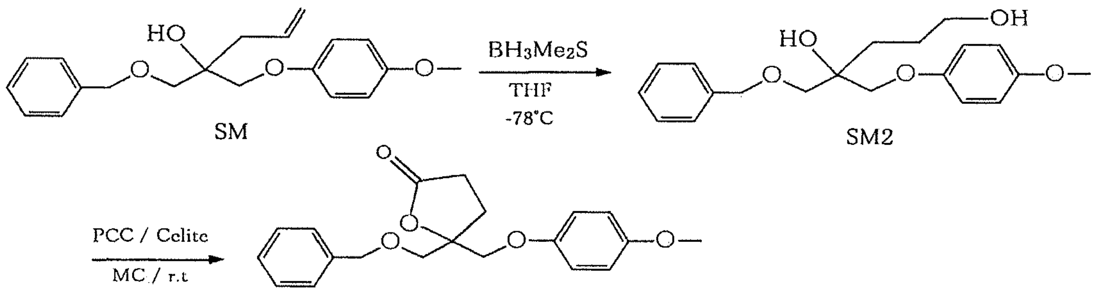


F. As shown in Reaction 1f below, 2.94g (8.59 mmol) of the product(SM) of Reaction 1e was dissolved in 70 ml of acetonitrile mixed solution (acetonitrile (ACN) / $H_2O$ = 8:2 (volume ratio) mixture). Then, it was cooled to 0 °C. 14.2g(25.76 mmole) of ceric ammonium nitrate(CAN) was added at the same temperature. The reaction was confirmed by TLC (EA:Hex=1:2). When the reaction was completed, a saturated sodium hydrogen carbonate solution (Saturated $NaHCO_3$ aq. Soln.) was added to the reaction solution to quench the reaction. The reaction mixture was heated to room temperature and extracted with ethyl acetate(EA)/$H_2O$. After removing moisture with magnesium sulfate ($MgSO_4$), it was concentrated. It was obtained 2.8g of the target compound (yield: 137.9%).

[Reaction 1f]

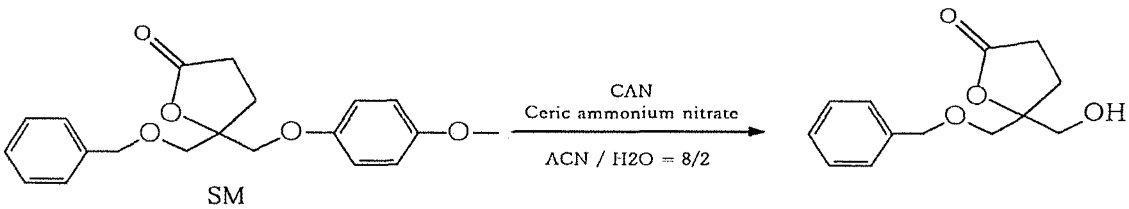


G. As shown in Reaction 1g below, 5.88g (22.945 mmole) of palmitic acid and 2.88ml (22.945mmole) of pivaloyl chloride were added in 58.8ml of Methylene chloride (MC) and it was cooled to 0 °C. While maintaining the same temperature, 7.4 ml (52.95 mmol) of triethylamine (TEA) was slowly added dropwise, followed by stirring at the same temperature for 30 minutes. 1.47g (17.65mmloe) of the product(SM) of Reaction 1f was added, and 216mg (1.765mmole) of 4-dimethylaminopyridine(DMAP) was added, and then the temperature was raised to room temperature. Then, it was stirred at the same temperature for 2 hours. The reaction was confirmed by TLC(EA:Hex=1:2). When the reaction was completed, the reaction solution was extracted with an aqueous potassium hydroxide solution(aq. KOH soln.)/methylene chloride(MC), and extracted with a hydrochloric acid solution(HCl soln.)/methylene chloride(MC), and then concentrated. The concentrate was purified with a flash column(eluent: ethyl acetate (EA): hexane(Hex) = 1:4:5(volume ratio) mixture) to obtain 1.9g of the target compound(yield: 22.6%).
Alternatively, SM (2.8g, 11.85mmole, 1eq.)/Palmitic acid(1.3eq.)/N,N'-dicyclohexylcarbodiimide (DCC, 1.3eq.)/DMAP(0.1eq)/MC (10 times of Palmitic acid) was reacted. The reaction was confirmed by TLC (EA:Hex=1:2). When the reaction was complete, the solvent was concentrated. Then, it was filtered after slurrying with an appropriate amount of hexane, and concentrated. The concentrate was purified with a flash column (eluent: ethyl acetate(EA): hexane(Hex) = 1:7(volume ratio) mixture) to obtain 3.66g of the target compound(yield: 65%).

[Reaction 1g]

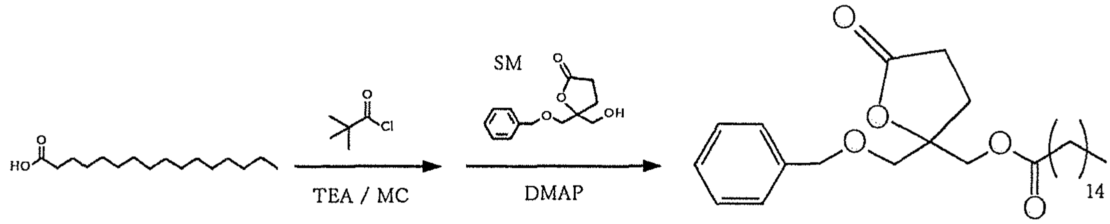


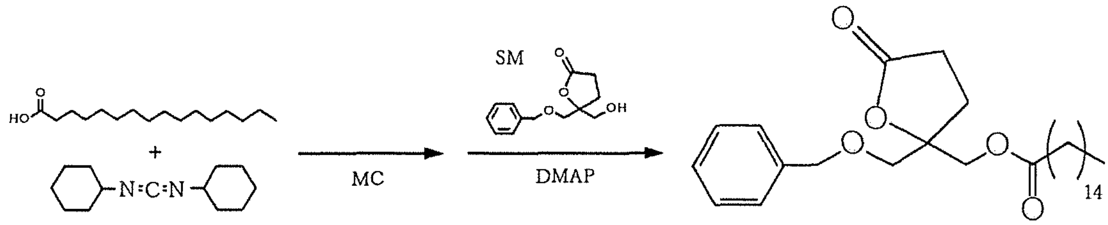


H. As shown in Reaction 1h below, 3.66g (7.71 mmol) of the product(SM) of Reaction 1g was added in 38 ml of methylene chloride(MC). Then, after bubbling with nitrogen gas ($N_2$), it was cooled to -78 °C. While maintaining the same temperature, 23 ml (23.11 mmol) of boron trichloride (1M in MC, $BCl_3$) was slowly added dropwise, followed by stirring at the same temperature for 2 hours. The reaction was confirmed by TLC (EA:Hex=1:1). When the reaction was completed, a saturated sodium hydrogen carbonate solution(Saturated $NaHCO_3$ aq. Soln.) was added to the reaction solution to quench the reaction. After extraction with methylene chloride(MC)/$H_2O$, it was concentrated. The concentrate was purified with a flash column(eluent: ethyl acetate(EA): hexane(Hex) = 1:2(volume ratio) mixture) to obtain 2.68g of the target compound(yield: 90%).

[Reaction 1h]

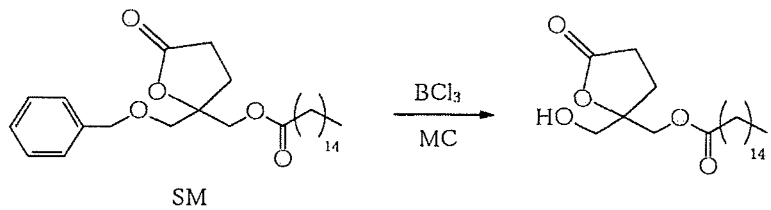


I. As shown in Reaction 1i below, 100g (0.26mmole) of the product(SM) of Reaction 1h, 72.5mL (0.52mmole) of triethylamine(TEA) and 3.17mg (0.026mmole) of 4-dimethylaminopyridine(DMAP) were added in 10 ml of methylene chloride(MC) and stirred at room temperature for 30 minutes. 24mL(0.338mmole) of acetyl chloride was slowly added dropwise, and it was stirred at the same temperature for 2 hours. The reaction was confirmed by TLC(EA:Hex=1:2). When the reaction was completed, the reaction solution was extracted with an aqueous potassium hydroxide solution(aq. KOH soln.)/methylene chloride(MC), and extracted with a hydrochloric acid solution(HCl soln.)/methylene chloride(MC), and then concentrated. The concentrate was purified with a flash column(eluent: ethyl acetate(EA): hexane(Hex) = 1:3(volume ratio) mixture) to obtain 87.4mg of the target compound(yield: 78.8%).

[Reaction 1i]

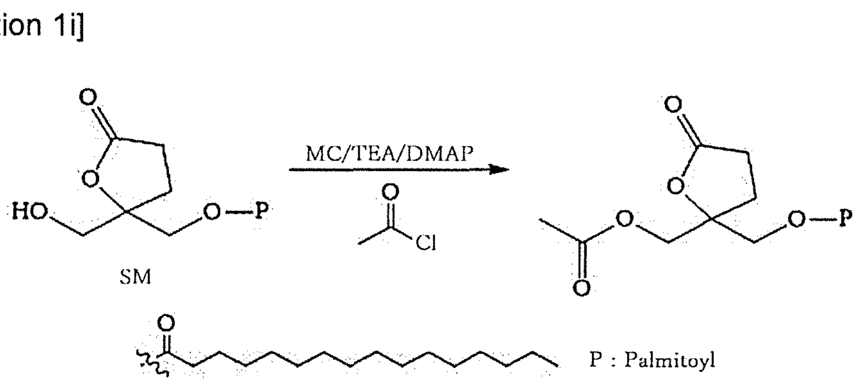

[Example 2] Synthesis of diacylglycerol lactone compound(EC-A51)

A. As shown in Reaction 2a below, 350ml of pyridine, 10g (111.01mmole) of dihydroxyacetone and 3.53g (28.86mmole) of 4-dimethylaminopyridine(DMAP) were dissolved. Then, after bubbling with nitrogen gas($N_2$), it was cooled to 0 °C. While maintaining the same temperature, 30.51 g(111.01mmole) of tert-butyl(chloro)diphenylsilane (TBDPSCl) was slowly added dropwise. The reaction was stirred at the same temperature for 15 minutes, and heated to 25 to 30 °C. It was stirred for 16 hours. The reaction was confirmed by TLC(PE:EA=10:1). When the reaction was completed, extraction was carried out 4 times with ethyl acetate(EA)/ purified water. The organic layer is sequentially washed with 1M-hydrochloric acid and brine soln., and the organic layer is dehydrated with sodium sulfate($Na_2SO_4$) and concentrated. The concentrate was purified with a flash column(eluent: Petroleum ether(PE): ethyl acetate(EA) = 50:1(volume ratio) mixture) to obtain 7g of the target compound(yield: 19.2%).

[Reaction 2a]

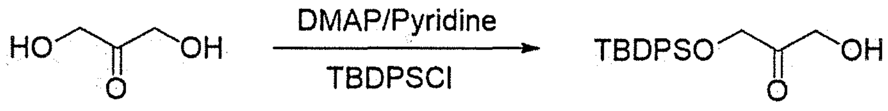


SM

B. As shown in Reaction 2b below, 361.21 mg (4.57mmole) of pyridine, 300mg (0.9133mmole) of the product(SM) of Reaction 2a and 16.74mg (0.13699mmole) of 4-dimethylaminopyridine(DMAP) were dissolved in 10ml of methylene chloride(MC). Then, after bubbling with nitrogen gas($N_2$), it was cooled to 0 °C. While maintaining the same temperature, 78.86mg (1mmole) of acetyl chloride was slowly added dropwise. The reaction was stirred at the same temperature for 5 minutes, and heated to 25 to 30 °C. It was stirred for 4 hours. The reaction was confirmed by TLC(PE:EA=5:1). When the reaction was completed, ice water was added to the reaction and stirred for 5 minutes. It was extracted 4 times with ethyl acetate(EA)/ purified water. The organic layer is sequentially washed with 1M-hydrochloric acid and brine soln., and the organic layer is dehydrated with sodium sulfate($Na_2SO_4$) and concentrated. The concentrate was purified with a flash column(eluent: Petroleum ether(PE): ethyl acetate(EA) = 50:1(volume ratio) mixture) to obtain 200mg of the target compound(yield: 59.1%).

[Reaction 2b]

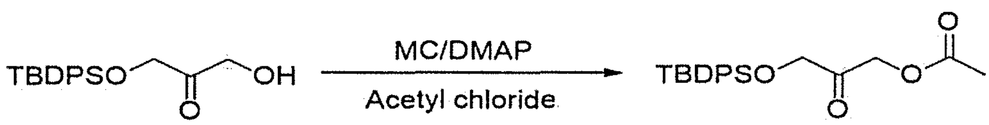


SM

C. As shown in Reaction 2c below, 250mg (0.6748mmole) of the product(SM) of Reaction 2b was dissolved in 2ml of tetrahydrofuran (THF). Then, after bubbling with nitrogen gas($N_2$), it was cooled to -70 °C. While maintaining the same temperature, 337.3mL (0.6748mmole) of allylmagnesium chloride(2M in THF) was slowly added dropwise. The reaction was stirred at the same temperature for 15 minutes and heated to 25 to 30 °C. It was stirred for 3 hours. The reaction was confirmed by TLC (PE:EA=10:1). When the reaction was completed, ice water was added to the reaction and stirred for 5 minutes. It was extracted 4 times with ethyl acetate(EA)/ purified water. The organic layer is sequentially washed with 1M-hydrochloric acid and brine soln., and the organic layer is dehydrated with sodium sulfate ($Na_2SO_4$) and concentrated. The concentrate was purified with a flash column(eluent: Petroleum ether(PE): ethyl acetate(EA) = 50:1 (volume ratio) mixture) to obtain 100mg of the target compound (yield: 28.74%).

[Reaction 2c]

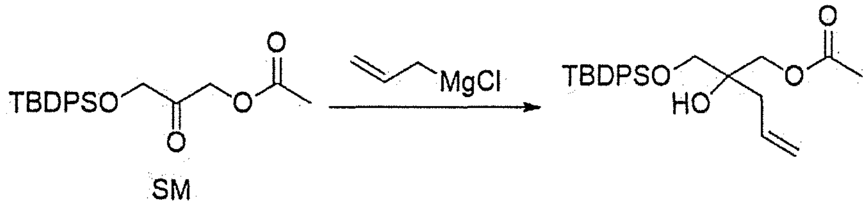


D. As shown in Reaction 2d below, 2g (4.85mmole) of the product(SM) of Reaction 2c was dissolved in 50ml of tetrahydrofuran(THF). Then, after bubbling with nitrogen gas ($N_2$), it was cooled to -78 °C. While maintaining the same temperature, 970mL (9.7mmole) of boranedimethylsulfide(10M in THF) was slowly added dropwise. The reaction was stirred at the same temperature for 30 minutes and heated to 25 to 35 °C. It was stirred for 15.5 hours. The reaction was confirmed by TLC(PE:EA=10:1). When the reaction was completed, 80 ml of methanol was added to quench the reaction. The organic layer was concentrated to obtain 2.5g of the target compound. This reaction product was used directly in the next reaction.

[Reaction 2d]

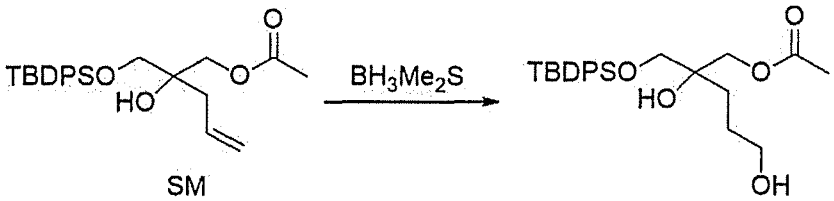


E. As shown in Reaction 2e below, 110mg (0.25545mmole) of the product(SM) of Reaction 2d was dissolved in 10ml of methylene chloride(MC). Then, after bubbling with nitrogen gas($N_2$), it was heated to 25 to 30 °C. While maintaining the same temperature, 550.65mg (2.55mmole) of pyridinium chlorochromate(PCC) was added to one portion. The reaction solution was stirred at the same temperature for 36 hours. The reaction was confirmed by TLC(PE:EA=2:1). When the reaction was completed, extraction was carried out 4 times with methylene chloride(MC)/ purified water. The organic layer was washed with and brine soln., and the organic layer is dehydrated with sodium sulfate($Na_2SO_4$) and concentrated. The concentrate was purified with a flash column(eluent: Petroleum ether(PE): ethyl acetate(EA) = 5:1 (volume ratio) mixture) to obtain 60mg of the target compound(yield: 44.05%).

[Reaction 2e]

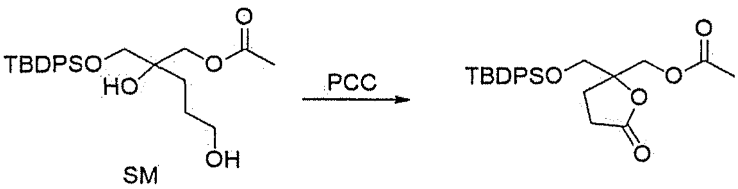


F. As shown in Reaction 2f below, 200mg(0.46885mmole) of the product(SM) of Reaction 2e was dissolved in 2ml of tetrahydrofuran(THF). Then, after bubbling with nitrogen gas($N_2$), it was heated to 25 to 30 °C. While maintaining the same temperature, 609.51 mL (1.3eq.) of tetrabutyl ammoniumfluoride(1M in THF, TBAF) was added to one portion. The reaction solution was stirred at the same temperature for 2 hours. The reaction was confirmed by TLC(PE:EA=10:1). When the reaction was completed, the reaction solution was filtered and concentrated. The concentrate was purified with a flash column(eluent: Petroleum ether(PE): ethyl acetate(EA) = 10:1 (volume ratio) mixture) to obtain 60mg of the target compound (yield: 54.4%).

[Reaction 2f]

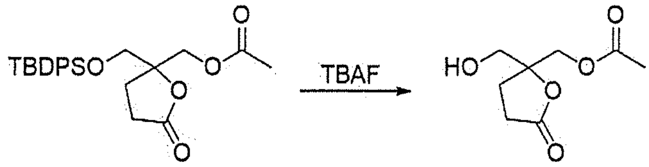


SM

G. As shown in Reaction 2g below, 19.96mg (0.10608mmole) of the product(SM) of Reaction 2f and 35mg (0.1248mmole) of linoleic acid were dissolved in 2ml of methylene chloride(MC). Then, after bubbling with nitrogen gas($N_2$), it was heated to 25 to 35 °C. While maintaining the same temperature, 30.9mg (0.14976mmole) of N,N'-dicyclohexylcarbodiimide (DCC) and 3.05mg (0.02496mmole) of 4-dimethylaminopyridine (DMAP) were added to one portion. The reaction was stirred at the same temperature for 16 hours. The reaction was confirmed by TLC(PE:EA=10:1). When the reaction was completed, ice water was added to the reaction solution and stirred for 5 minutes. It was extracted 4 times with ethyl acetate(EA)/ purified water. The organic layer was washed with brine soln. The organic layer is dehydrated with sodium sulfate($Na_2SO_4$) and concentrated. The concentrate was purified with a flash column(eluent: Petroleum ether(PE): ethyl acetate(EA) = 10:1 (volume ratio) mixture) to obtain 20 mg of the target compound (yield: 33.79%).

[Reaction 2g]

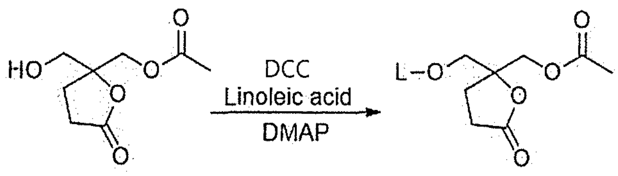


SM

L : Linoleoyl

[Example 3] Synthesis of diacylglycerol lactone compound(EC-A52)

A. As shown in Reaction 3a below, 350ml of pyridine, 10g (111.01mmole) of dihydroxyacetone and 3.53g (28.86mmole) of 4-dimethylaminopyridine(DMAP) were dissolved. Then, after bubbling with nitrogen gas($N_2$), it was cooled to 0 °C. While maintaining the same temperature, 30.51 g (111.01mmole) of tert-butyl(chloro)diphenylsilane (TBDPSCI) was slowly added dropwise. The reaction solution was stirred at the same temperature for 15 minutes and heated to 25 to 30 °C. It was stirred for 16 hours. The reaction was confirmed by TLC(PE:EA=10:1). When the reaction was completed, extraction was carried out 4 times with ethyl acetate(EA)/ purified water. The organic layer is sequentially washed with 1M-hydrochloric acid and brine soln., and the organic layer is dehydrated with sodium sulfate ($Na_2SO_4$) and concentrated. The concentrate was purified with a flash column(eluent: Petroleum ether(PE): ethyl acetate(EA) = 50:1 (volume ratio) mixture) to obtain 7g of the target compound(yield: 19.2%).

[Reaction 3a]

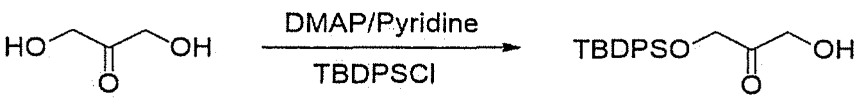


SM

B. As shown in Reaction 3b below, 6g (76.1mmole) of pyridine, 5g (15.22mmole) of the product(SM) of Reaction 3a and 185.94mg(1.52mmole) of 4-dimethylaminopyridine (DMAP) were dissolved in 150ml of methylene chloride(MC). Then, after bubbling with nitrogen gas(N2), it was heated to 25 to 35 °C. While maintaining the same temperature, 4.18g (15.22mmole) of palmitoyl chloride was slowly added dropwise. The reaction solution was stirred

at the same temperature for 5 minutes, and heated to 25 to 35 °C. It was stirred for 16 hours. The reaction was confirmed by TLC(PE:EA=10:1). When the reaction was completed, ammonium chloride aqueous solution ($NH_4Cl$ soln.) was added to the reaction solution to quench the reaction. Then, it was extracted 3 times with ethyl acetate(EA)/ purified water. The organic layer was washed with brine soln. The organic layer was dehydrated with sodium sulfate($Na_2SO_4$) and concentrated. The concentrate was purified with a flash column(eluent: Petroleum ether(PE): ethyl acetate(EA) = 50:1(volume ratio) mixture) to obtain 4g of the target compound(yield: 41.72%).

[Reaction 3b]

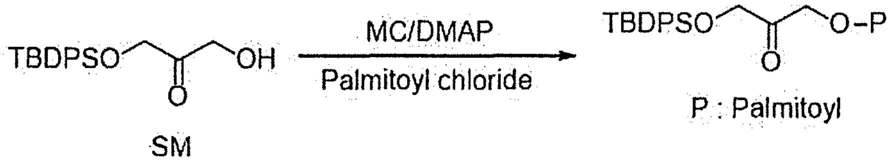


C. As shown in Reaction 3c below, 3.15g (5.55mmole) of the product(SM) of Reaction 3b was dissolved in 50ml of tetrahydrofuran(THF). Then, after bubbling with nitrogen gas($N_2$), it was cooled to 0 °C. While maintaining the same temperature, 1.61g (11.1 mmole) of allylmagnesium bromide was slowly added dropwise. The reaction solution was stirred at the same temperature for 15 minutes and heated to 25 to 30 °C. It was stirred for 16 hours. The reaction was confirmed by TLC(PE:EA=10:1). When the reaction was completed, it was extracted 3 times with ethyl acetate(EA)/ purified water. The organic layer was washed with brine soln. The organic layer was dehydrated with sodium sulfate ($Na_2SO_4$) and concentrated. The concentrate was purified with a flash column(eluent: Petroleum ether(PE): ethyl acetate(EA) = 20:1(volume ratio) mixture) to obtain 1.5g of the target compound(yield: 44.32%).

[Reaction 3c]

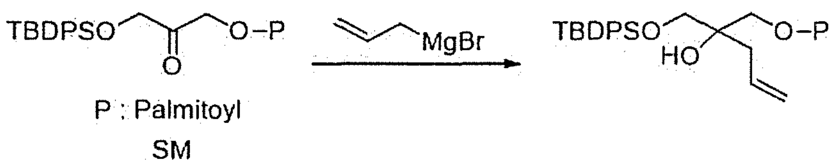


D. As shown in Reaction 3d below, 1.2g(2.91 mmole) of the product(SM) of Reaction 3c was dissolved in 30ml of tetrahydrofuran(THF). Then, after bubbling with nitrogen gas($N_2$), it was cooled to -78 °C. While maintaining the same temperature, 662.87mg (8.73mmole) of boranedimethylsulfide was slowly added dropwise. The reaction solution was stirred at the same temperature for 1 hour and heated to 25 to 35 °C. It was stirred for 15 hours. The reaction was confirmed by TLC(PE:EA=10:1). When the reaction was completed, 80ml of methanol was added to the reaction solution to quench the reaction. The organic layer was concentrated to obtain 1.3g of the target compound. This reaction product was used directly in the next reaction.

[Reaction 3d]

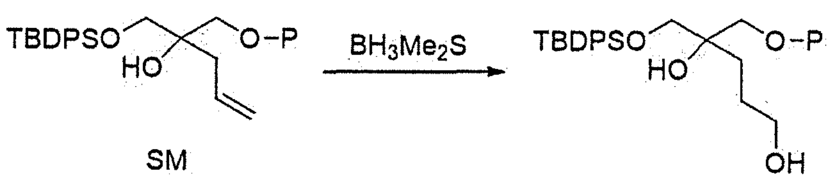


E. As shown in Reaction 3e below, 150mg (0.23924mmole) of the product(SM) of Reaction 3d was dissolved in 15ml of methylene chloride(MC). Then, after bubbling with nitrogen gas($N_2$), it was heated to 25 to 30 °C. While maintaining the same temperature, 361mg (1.67mmole) of pyridinium chlorochromate(PCC) was added to one portion. The reaction solution was stirred at the same temperature for 48 hours. The reaction was confirmed by TLC (PE:EA=5:1). When the reaction was completed, the reaction product was concentrated to obtain 150 mg of the target compound.

[Reaction 3e]

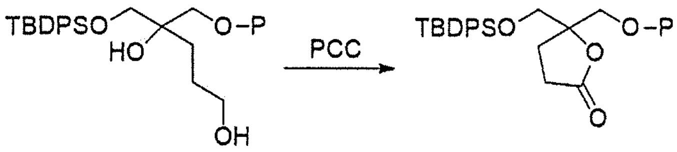


SM

F. As shown in Reaction 3f below, 1.5g (2.41mmole) of the product(SM) of Reaction 3e was dissolved in 5ml of tetrahydrofuran(THF). Then, after bubbling with nitrogen gas($N_2$), it was heated to 25 to 35 °C. While maintaining the same temperature, 3.13mL (1.3eq.) of tetrabutyl ammoniumfluoride(1 M in THF, TBAF) was added to one portion. The reaction solution was stirred at the same temperature for 2 hours. The reaction was confirmed by TLC(PE:EA=5:1). When the reaction was completed, it was extracted 3 times with ethyl acetate(EA)/ purified water. The organic layer was washed with brine soln., and the organic layer was dehydrated with sodium sulfate($Na_2SO_4$) and concentrated. The concentrate was purified with a flash column(eluent: Petroleum ether (PE): ethyl acetate(EA) = 5:1(volume ratio) mixture) to obtain 250mg of the target compound (yield: 24.28%).

[Reaction 3f]

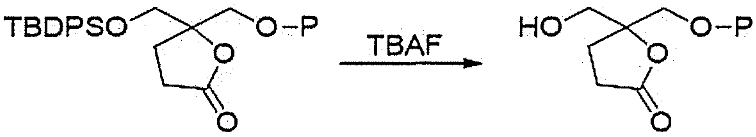


SM

G. As shown in Reaction 3g below, 250mg (0.65011mmole) of the product(SM) of Reaction 3f and 182.32mg (0.65011mmole) of linoleic acid were dissolved in 5ml of methylene chloride(MC). Then, after bubbling with nitrogen gas ($N_2$), it was heated to 25 to 35 °C. While maintaining the same temperature, 160.96mg (0.78013mmole) of N,N'-Dicyclohexylcarbodiimide(DCC) and 7.94mg (0.06501mmole) of 4-dimethylaminopyridine(DMAP) were added to one portion. The reaction solution was stirred at the same temperature for 16 hours. The reaction was confirmed by TLC (PE:EA=5:1). When the reaction was completed, the reaction solution was filtered and concentrated. The concentrate was purified with a flash column(eluent: Petroleum ether(PE): ethyl acetate(EA) = 5:1(volume ratio) mixture) to obtain 100mg of the target compound(yield: 22.59%).

[Reaction 3g]

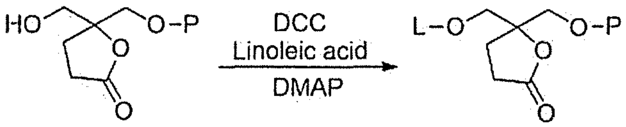


SM

L : Linoleoyl

[Example 4] Synthesis of diacylglycerol lactone compound(EC-A115)

A. As shown in Reaction 4a below, 65mg (0.275mmole) of the product(SM) of Reaction 1f, 77mL (0.55mmole) of triethylamine(TEA) and 3.36mg (0.0275mmole) of 4-dimethylaminopyridine (DMAP) were added in 2ml of methylene chloride(MC), and stirred, followed by stirring at room temperature for 30 minutes. 31.2mL (0.358mmole) of propionyl chloride was slowly added dropwise in the reaction solution and stirred at room temperature for 2 hours. The reaction was confirmed by TLC(EA:Hex=1:2). When the reaction was completed, extraction was carried out with 0.1N-hydrochloric acid aqueous solution(c-HCl soln.)/methylene chloride(MC). The concentrate was purified with a flash column(eluent: ethyl acetate(EA): hexane(Hex) = 1:3 (volume ratio) mixture) to obtain 51.2mg of the target compound(yield: 63.7%).

[Reaction 4a]

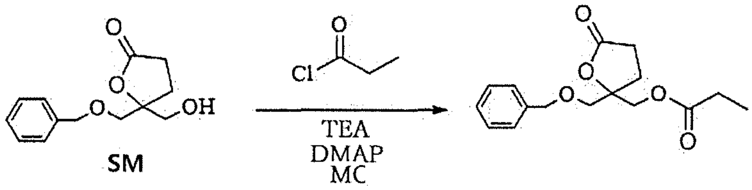


B. As shown in Reaction 4b below, 51.2mg (0.175mmole) of the product(SM) of Reaction 4a was dissolved in 1ml of methylene chloride(MC). Then, after bubbling with nitrogen gas($N_2$), it was cooled to -78 °C. 0.53ml(0.525mmole) of boron trichloride(1 M in MC, $BCl_3$) was slowly added dropwise in the coolant, and stirred at the same temperature for 2 hours. The reaction was confirmed by TLC(EA:Hex=1:2). When the reaction was completed, a saturated sodium hydrogen carbonate solution (Saturated $NaHCO_3$ soln.) was added to the reaction solution to quench the reaction, and the temperature was raised to room temperature. It was extracted with methylene chloride(MC) and concentrated. The concentrate was purified with a flash column(eluent: ethyl acetate(EA): hexane(Hex) = 2:1 (volume ratio) mixture) to obtain 25.6mg of the target compound(yield: 72.3%).

[Reaction 4b]

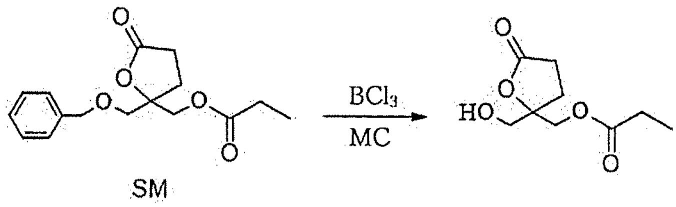


C. As shown in Reaction 4c below, 35.4mg (0.126mmole) of linoleic acid and 15.2mL(0.1236mmole) of pivaloyl chloride were added in 1 ml of methylene chloride(MC) and it was cooled to 0 to 5 °C. 34.5mL (0.2472mmole) of triethylamine (TEA) was slowly added dropwise and stirred at the same temperature for 30 minutes. 25mg(0.1236mmole) of the product(SM) of Reaction 4b and 1.5mg(0.0124mmole) of 4-dimethylaminopyridine(DMAP) were added and stirred at room temperature for overnight. The reaction was confirmed by TLC(EA:Hex=1:2). When the reaction was completed, it was extracted with 0.1N-potassium hydroxide solution(KOH soln.)/methylene chloride(MC). Then, the extractive solution was extracted with 0.07N-hydrochloric acid solution(c-HCl soln.)/methylene chloride(MC) and then concentrated after removing moisture with magnesium sulfate ($MgSO_4$). The concentrate was purified with a flash column(eluent: ethyl acetate(EA): hexane(Hex) = 1:3.5(volume ratio) mixture) to obtain 29.9mg of the target compound(yield: 52%).

[Reaction 4c]

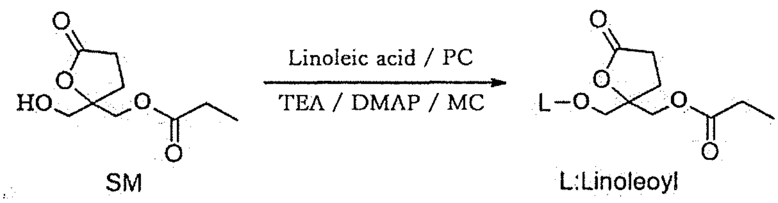


[Example 5] Synthesis of diacylglycerol lactone compound(EC-A116)

A. As shown in Reaction 5a below, 150mg (0.635mmole) of the produc(SM)t of Reaction 1f and 0.18 ml(1.27 mmole) of triethylamine(TEA) were added in 6.35ml of methylene chloride(MC) and stirred at room temperature for 30 minutes. 86.2mL(0.825mmole) of butyryl chloride was slowly added dropwise in the reaction solution and stirred at room temperature for 3 hours. The reaction was confirmed by TLC(EA:Hex=1:2). When the reaction was completed, extraction was carried out with 0.1N-hydrochloric acid aqueous solution(c-HCl soln.)/methylene chloride(MC) and

concentrated. The concentrate was purified with a flash column(eluent: ethyl acetate(EA): hexane(Hex) = 1:3.5(volume ratio) mixture) to obtain 106mg of the target compound(yield: 54.5%).

[Reaction 5a]

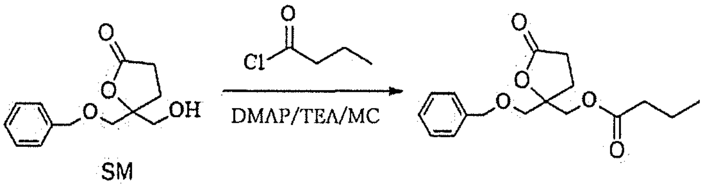


SM

B. As shown in Reaction 5b below, 106mg (0.346mmole) of the product(SM) of Reaction 5a was dissolved in 1ml of methylene chloride(MC). Then, after bubbling with nitrogen gas($N_2$), it was cooled to -78 °C. 1ml(1.038mmole) of boron trichloride(1M in MC, BCl3) was slowly added dropwise and stirred at the same temperature for 2 hours. The reaction was confirmed by TLC(EA:Hex=1:1). When the reaction was completed, a saturated sodium hydrogen carbonate solution(Saturated $NaHCO_3$ aq. Soln.) was added to the reaction solution to quench the reaction and it was heated to room temperature. It was extracted with methylene chloride(MC). The concentrate was purified with a flash column(eluent: ethyl acetate(EA): hexane(Hex) = 1:1(volume ratio) mixture) to obtain 52.8mg of the target compound(yield: 70.5%).

[Reaction 5b]

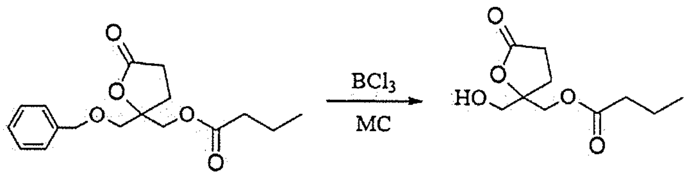


SM

C. As shown in Reaction 5c below, 70mg(0.249mmole) of linoleic acid and 30mL(0.24436mmole) of pivaloyl chloride were added in 1ml of methylene chloride(MC) and it was cooled to 0 to 5 °C. 68mL(0.488mmole) of triethylamine(TEA) was slowly added dropwise and stirred at the same temperature for 30 minutes. 52.8mg (0.244mmole) of the product(SM) of Reaction 5b and 3mg(0.0244mmole) of 4-dimethylaminopyridine(DMAP) were added in the reaction solution at the same temperature and stirred at the room temperature for overnight. The reaction was confirmed by TLC(EA:Hex=1:2). When the reaction was completed, it was extracted with 0.1N-potassium hydroxide solution (KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:3.5(volume ratio) mixture) to obtain 78.4mg of the target compound (yield: 67%).

[Reaction 5c]

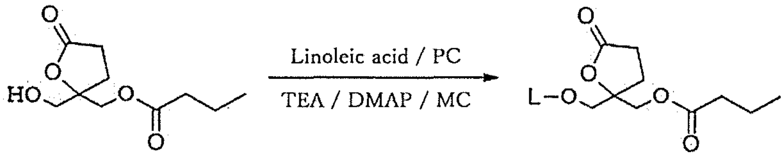


SM

L:Linoleoyl

[Example 6] Synthesis of diacylglycerol lactone compound(EC-A117)

A. As shown in Reaction 6a below, 150mg (0.635mmole) of the product(SM) of Reaction 1f and 0.18ml(1.27mmole) of triehtlyamine (TEA) were added in 2 ml of methylene chloride(MC) and stirred. Then It was stirred at room

temperature for 30 minutes. 0.1ml(0.825mmole) of valeroyl chloride was slowly added dropwise in the reaction solution and stirred at room temperature for 3 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:3.5(volume ratio) mixture) to obtain 89.1mg of the target compound(yield: 43.8%).

[Reaction 6a]

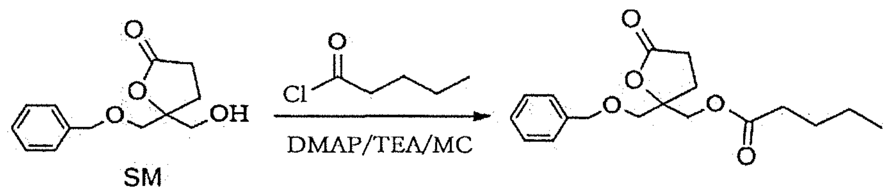


B. As shown in Reaction 6b below, 89.1mg(0.278mmole) of the product(SM) of Reaction 6a was dissolved in 1.4ml of methylene chloride(MC). Then, after bubbling with nitrogen gas($N_2$), it was cooled to -78 °C. 0.83ml(0.834mmole) of boron trichloride (1M in MC, $BCl_3$) was slowly added dropwise. The reaction solution was stirred at the same temperature for 1 hour. The reaction was confirmed by TLC(EA:Hex=1:1). When the reaction was completed, a saturated sodium hydrogen carbonate solution(Saturated $NaHCO_3$ aq. Soln.) was added to the reaction solution to quench the reaction and it was heated to room temperature. It was extracted with methylene chloride(MC) and then concentrated. The concentrate was purified with a flash column (eluent: ethyl acetate(EA): hexane(Hex) = 1:1 (volume ratio) mixture) to obtain 46.8mg of the target compound (yield: 73.1%).

[Reaction 6b]

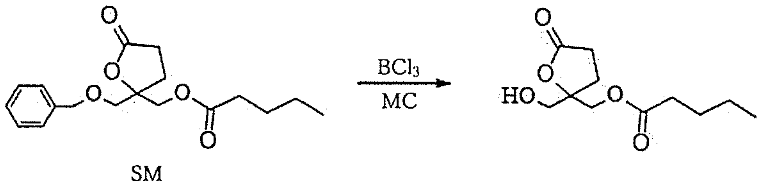


C. As shown in Reaction 6c below, 58.1mg (0.207mmole) of linoleic acid and 25mL (0.203mmole) of pivaloyl chloride was added in 1ml of methylene chloride(MC) and it was cooled to 0 to 5 °C. 57mL(0.406mmole) of triethylamine(TEA) was slowly added dropwise and stirred at the same temperature for 30 minutes. 46.8mg (0.203mmole) of the product(SM) of Reaction 6b and 2.5mg(0.0203mmole) of 4-dimethylaminopyridine(DMAP) were added at the same temperature and stirred at room temperature for overnight. The reaction was confirmed by TLC (EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:3.5(volume ratio) mixture) to obtain 42.5mg of the target compound(yield: 42.5%).

[Reaction 6c]

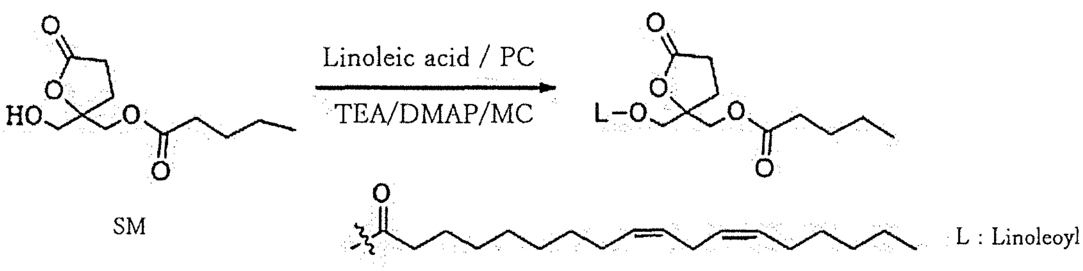

[Example 7] Synthesis of diacylglycerol lactone compound(EC-A118)

A. As shown in Reaction 7a below, 150mg (0.635mmole) of the product(SM) of Reaction 1f, 0.18ml (1.27mmole) of triethylamine(TEA) and 7.8mg(0.063mmole) of 4-dimethylaminopyridine(DMAP) were added in 2ml of methylene chloride (MC) and stirred. Then, it was stirred at room temperature for 30 minutes. 0.113mL (0.825mmole) of hexanoyl chloride was slowly added dropwise. The reaction solution was stirred at room temperature for 3 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column (eluent: ethyl acetate(EA): hexane(Hex) = 1:3.5 (volume ratio) mixture) to obtain 135.4mg of the product(yield: 63.7%).

[Reaction 7a]

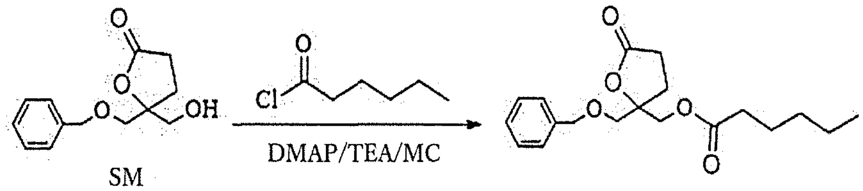


B. As shown in Reaction 7b below, 135.4mg (0.405mmole) of the product(SM) of Reaction 7a was dissolved in 2ml of methylene chloride(MC). Then, after bubbling with nitrogen gas($N_2$), it was cooled to -78 °C. 1.2ml(1.21mmole) of boron trichloride(1M in MC, $BCl_3$) was slowly added dropwise. The reaction solution was stirred at the same temperature for 2 hours. The reaction was confirmed by TLC(EA:Hex=1:1). When the reaction was completed, a saturated sodium hydrogen carbonate solution (Saturated $NaHCO_3$ aq. Soln.) was added to the reaction solution to quench the reaction, and it was heated to room temperature. It was extracted with methylene chloride(MC) and concentrated. The concentrate was purified with a flash column(eluent: ethyl acetate(EA): hexane(Hex) = 1:1(volume ratio) mixture) to obtain 70.5mg mg of the product(yield: 71.3%).

[Reaction 7b]

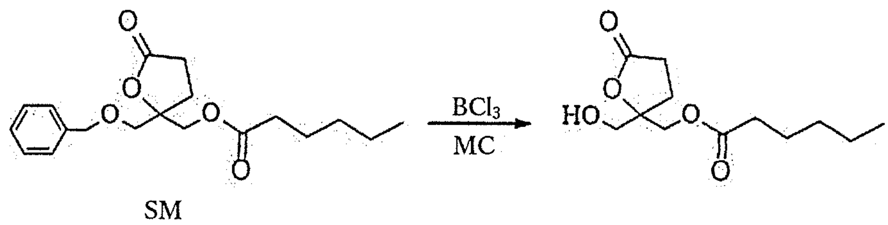


C. As shown in Reaction 7c below, 83mg (0.294mmole) of linoleic acid and 35.5mL(0.2886mmole) of pivaloyl chloride were added in 1ml of methylene chloride (MC). Then, It was cooled to 0 to 5 °C. 80mL(0.5772mmole) of triethylamine(TEA) was slowly added dropwise and stirred at the same temperature for 30 minutes. 70.5mg (0.2886mmole) of the product (SM) of Reaction 7b and 4.3mg(0.035mmole) of 4-dimethylaminopyridine(DMAP) were added at the same temperature and stirred at room temperature for overnight. The reaction was confirmed by TLC(EA:Hex=1:2). When the reaction was completed, it was extracted with 0.1N-potassium hydroxide solution (KOH soln.)/methylene chloride (MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4(volume ratio) mixture) to obtain 20mg of the target compound(yield: 13.7%).

[Reaction 7c]

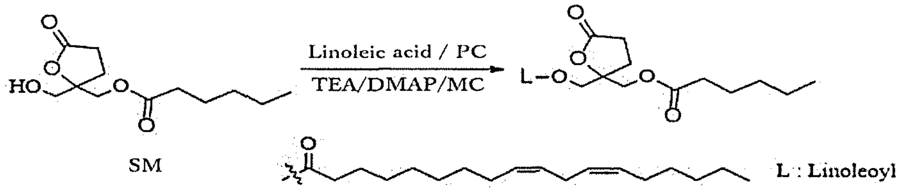


SM

[Example 8] Synthesis of diacylglycerol lactone compound(EC-A119)

A. As shown in Reaction 8a below, 150mg (0.635mmole) of the product(SM) of Reaction 1f, 0.18ml (1.27mmole) of triethylamine(TEA) and 7.8mg(0.063mmole) of 4-dimethylaminopyridine(DMAP) were added in 3ml of methylene chloride(MC) and stirred. Then, it was stirred at room temperature for 30 minutes. 0.13ml(0.825mmole) of heptanoyl chloride was slowly added dropwise and stirred at room temperature for 3 hours. The reaction was confirmed by TLC (EA:Hex=1:2). When the reaction was completed, it was extracted with 0.1N-hydrochloric acid solution(c-HCl soln.)/methylene chloride(MC) and then concentrated. The concentrate was purified with a flash column (eluent: ethyl acetate(EA): hexane(Hex) = 1:3.5(volume ratio) mixture) to obtain 126.1mg of the product (yield: 57%).

[Reaction 8a]

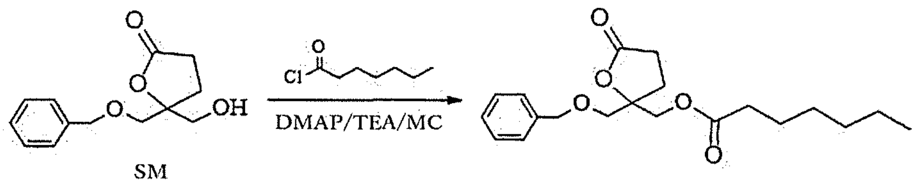


SM

B. As shown in Reaction 8b below, 126mg(0.362mmole) of the product(SM) of Reaction 8a was dissolved in 2ml of methylene chloride(MC). Then, after bubbling with nitrogen gas($N_2$), it was cooled to -78 °C. 1.1ml(1.08mmole) of boron trichloride(1M in MC, $BCl_3$) was slowly added dropwise. The reaction solution was stirred at the same temperature for 2 hours. The reaction was confirmed by TLC(EA:Hex=1:2). When the reaction was completed, extraction was carried out with 0.1N-hydrochloric acid aqueous solution(c-HCl soln.)/methylene chloride(MC) and concentrated. The concentrate was purified with a flash column(eluent: ethyl acetate(EA): hexane(Hex) = 1:1(volume ratio) mixture) to obtain 77.8mg of the product(yield: 83.2%).

[Reaction 8b]

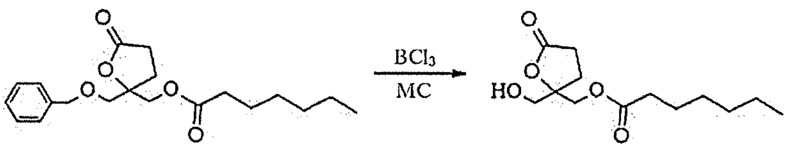


SM

C. As shown in Reaction 8c below, 86.2mg(0.3072mmole) of linoleic acid and 37mL (0.3012mmole) of pivaloyl chloride were added in 1ml of methylene chloride(MC). Then, It was cooled to 0 to 5 °C. 84mL (0.6024mmole) of triethylamine(TEA) was slowly added dropwise and stirred at the same temperature for 30 minutes. 77.8mg (0.3012mmole) of the product(SM) of Reaction 8b and 3.7mg (0.0301 mmole) of 4-dimethylaminopyridine(DMAP) were added at the same temperature and stirred at room temperature for overnight. The reaction was confirmed by TLC (EA:Hex=1:2). When the reaction was completed, it was extracted with 0.1N-potassium hydroxide solution (KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:3.5 (volume ratio) mixture) to obtain 75.1mg of the target compound(yield: 47.9%).

[Reaction 8c]

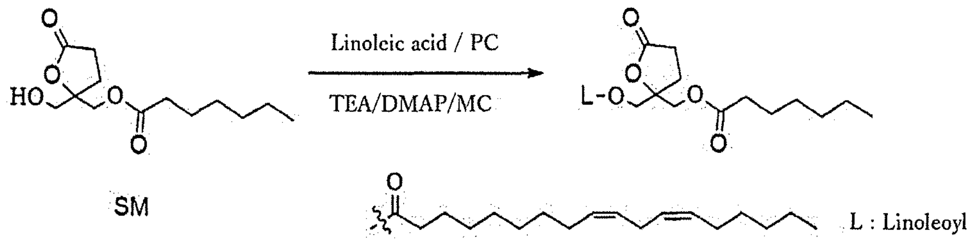


[Example 9] Synthesis of diacylglycerol lactone compound(EC-A120)

A. As shown in Reaction 9a below, 150mg (0.635mmole) of the product(SM) of Reaction 1f, 0.18ml (1.27mmole) of triethylamine(TEA) and 7.8mg(0.063mmole) of 4-dimethylaminopyridine(DMAP) were added in 2ml of methylene chloride(MC) and stirred. Then, it was stirred at room temperature for 30 minutes. 0.16ml (0.825mmole) of nonanoyl chloride was slowly added dropwise. The reaction solution was stirred at room temperature for 3 hours. The reaction was confirmed by TLC (EA:Hex=1:2). When the reaction was completed, it was extracted with 0.1N-hydrochloric acid solution(c-HCl soln.)/methylene chloride(MC) and then concentrated. The concentrate was purified with a flash column (eluent: ethyl acetate(EA): hexane(Hex) = 1:3.5 (volume ratio) mixture) to obtain 154mg of the target compound(yield: 64%).

[Reaction 9a]

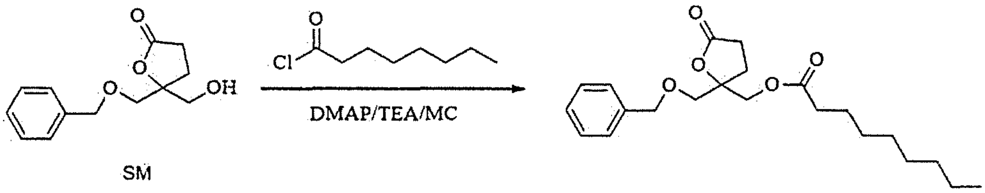


B. As shown in Reaction 9b below, 154mg(0.41mmole) of the product(SM) of Reaction 9a was dissolved in 2ml of methylene chloride(MC). Then, after bubbling with nitrogen gas($N_2$), it was cooled to -78 °C. 1.21ml(1.227mmole) of boron trichloride(1M in MC, $BCl_3$) was slowly added dropwise. The reaction solution was stirred at the same temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:1(volume ratio) mixture) to obtain 99.7mg of the target compound(yield: 85%).

[Reaction 9b]

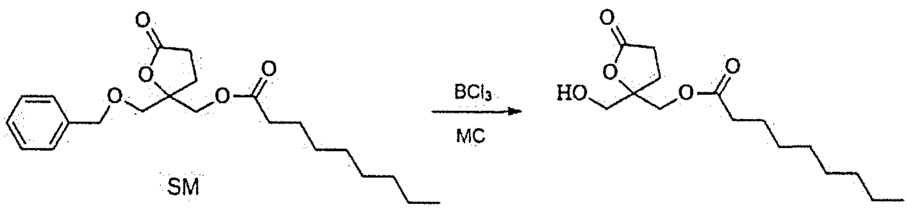


C. As shown in Reaction 9c below, 100mg (0.355mmole) of linoleic acid and 43mL (0.355mmole) of pivaloyl chloride were added in 1ml of methylene chloride(MC). Then, it was cooled to 0 to 5 °C. 97mL(0.696mmole) of triethylamine(TEA) was slowly added dropwise. The reaction solution was stirred at the same temperature for 30 minutes. 99.7mg(0.348mmole) of the product(SM) of Reaction 9b and 4.3mg(0.035mmole) of 4-dimethylaminopyridine(DMAP) were added at the same temperature and stirred at room temperature for overnight. The reaction was confirmed by TLC(EA:Hex=1:2). When the reaction was completed, it was extracted with 0.1N-potassium hydroxide

solution(KOH soln.)/methylene chloride(MC). The extract solution was extracted with 0.07N-hydrochloric acid solution (c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:3.5 (volume ratio) mixture) to obtain 87.1mg of the target compound(yield: 45.6%).

[Reaction 9c]

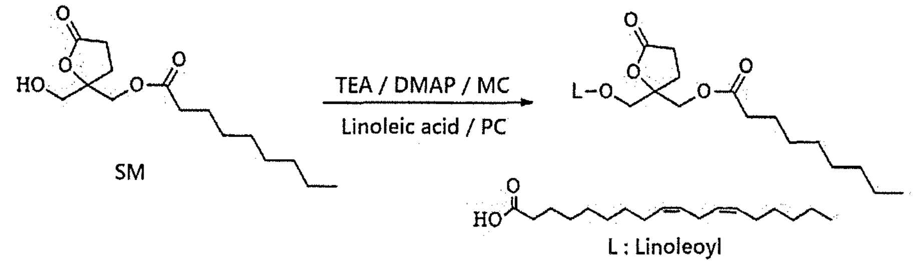


[Example 10] Synthesis of diacylglycerol lactone compound(EC-A121)

A. As shown in Reaction 10a below, 165.264mg (0.825mmole) of lauric acid and 43mL(0.825mmole) of pivaloyl chloride were added in 2ml of methylene chloride (MC). Then, It was cooled to 0 °C. 0.27ml (3mmole) of triethylamine (TEA) was slowly added dropwise. The reaction solution was stirred at the same temperature for 30 minutes. 150mg (0.635mmole) of the product (SM) of Reaction 1f and 7.8mg (0.0635mmole) of 4-dimethylaminopyridine (DMAP) were added at the same temperature and stirred at room temperature for 2 hours. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution (c-HCl soln.)/methylene chloride (MC) and then concentrated after removing moisture with magnesium sulfate ($MgSO_4$). The concentrate was purified with a flash column(eluent: ethyl acetate(EA): hexane(Hex) = 1:4 (volume ratio) mixture) to obtain 159.1mg of the target compound(yield: 59.8%).

[Reaction 10a]

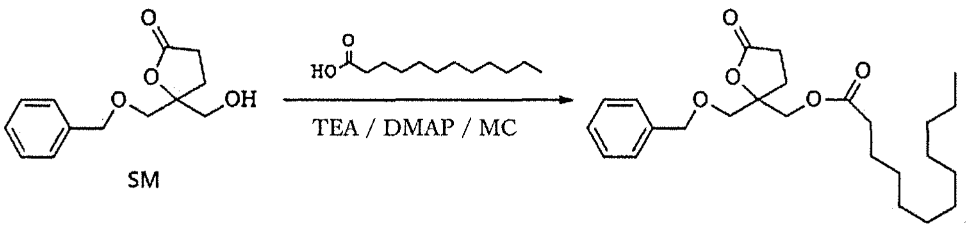


B. As shown in Reaction 10b below, 159mg(0.3798mmole) of the product(SM) of Reaction 10a was dissolved in 1.9ml of methylene chloride(MC). Then, after bubbling with nitrogen gas($N_2$), it was cooled to -78 °C. 1.14ml(1.14mmole) of boron trichloride(1M in MC, $BCl_3$) was slowly added dropwise. The reaction solution was stirred at the same temperature for 2 hours. The reaction was confirmed by TLC (EA:Hex=1:2). When the reaction was completed, 0.1N-hydrochloric acid solution(c-HCl soln.)/methylene chloride(MC) and then concentrated. It was obtained 113.6mg of the target compound (yield: 91.1%).

[Reaction 10b]

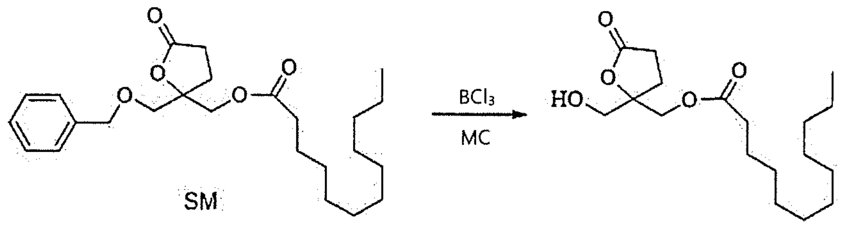


C. As shown in Reaction 10c below, 99mg (0.3528mmole) of linoleic acid and 42.56mL (0.3459mmole) of pivaloyl chloride were added in 1ml of methylene chloride (MC). Then, It was cooled to 0 °C. 0.15ml (1.0377mmole) of triethylamine(TEA) was slowly added dropwise. The reaction solution was stirred at the same temperature for 30 minutes. 113.6mg (0.3459mmole) of the product(SM) of Reaction 10b and 4 mg(0.0346mmole) of 4-dimethylaminopyridine(DMAP) were added at the same temperature and stirred at room temperature for overnight. The reaction was confirmed by TLC (EA:Hex=1:2). When the reaction was completed, it was extracted with 0.1N-potassium hydroxide solution (KOH soln.)/Methylene chloride (MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column (eluent: ethyl acetate(EA): hexane(Hex) = 1:5 (volume ratio) mixture) to obtain 128mg of the target compound (yield: 62.6%).

[Reaction 10c]

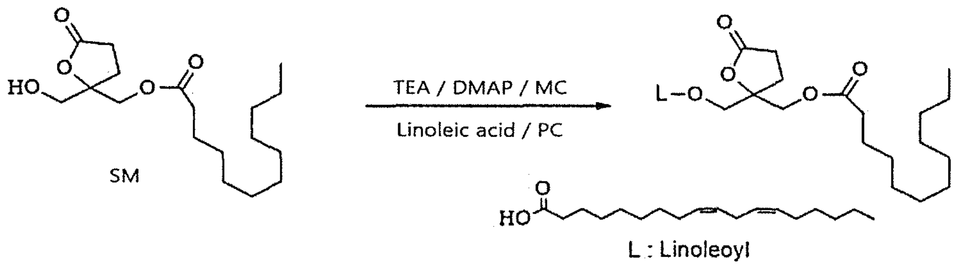


[Example 11] Synthesis of diacylglycerol lactone compound(EC-A122)

A. As shown in Reaction 10a below, 188.405mg (0.825mmole) of myristic acid and 43mL (0.825mmole) of pivaloyl chloride were added in 2ml of methylene chloride (MC). Then, It was cooled to 0 °C. 0.27ml (3mmole) of triethylamine(TEA) was slowly added dropwise. The reaction solution was stirred at the same temperature for 30 minutes. 150mg (0.635mmole) of the product(SM) of Reaction 1f and 7.8mg (0.0635mmole) of 4-dimethylaminopyridine(DMAP) were added at the same temperature and stirred at room temperature for 2 hours. The reaction was confirmed by TLC (EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4 (volume ratio) mixture) to obtain 190.7mg of the target compound(yield: 67.2%).

[Reaction 11a]

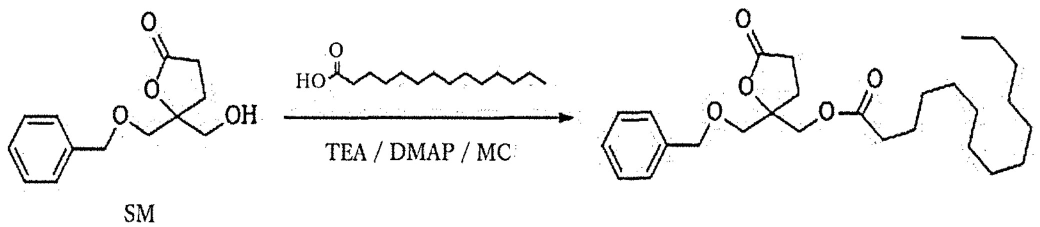

B. As shown in Reaction 11b below, 190.7mg (0.427mmole) of the product(SM) of Reaction 10a was dissolved in 2ml of methylene chloride(MC). Then, after bubbling with nitrogen gas($N_2$), it was cooled to -78 °C. 1.28ml (1.28mmole) of boron trichloride(1M in MC, $BCl_3$) was slowly added dropwise. The reaction solution was stirred at the same temperature for 2 hours. The reaction was confirmed by TLC (EA:Hex=1:2). When the reaction was completed, it was extracted with 0.1N-hydrochloric acid solution(c-HCl soln.)/methylene chloride(MC) and then concentrated. It was obtained 111.2mg of the target compound(yield: 73%).

[Reaction 11b]

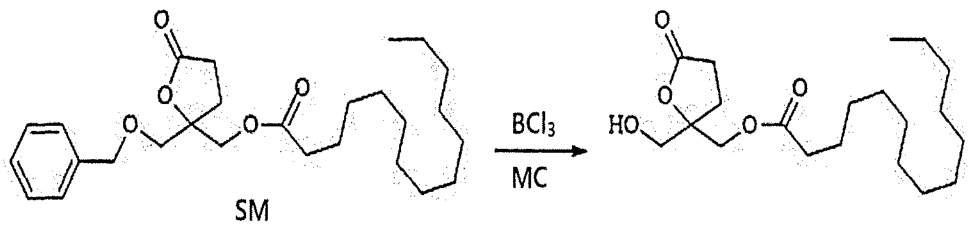


C. As shown in Reaction 11c below, 89mg (0.3176mmole) of linoleic acid and 38mL (0.3012mmole) of pivaloyl chloride were added in 1ml of methylene chloride(MC). Then, it was cooled to 0 °C. 94.5mL (0.9341mmole) of triethylamine(TEA) was slowly added dropwise. The reaction solution was stirred at the same temperature for 30 minutes. 111mg(0.3113mmole) of the product(SM) of Reaction 10b and 3.8mg (0.031mmole) of 4-dimethylaminopyridine(DMAP) were added at the same temperature and stirred at room temperature for overnight. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:5(volume ratio) mixture) to obtain 104.5mg of the target compound(yield: 54.2 %).

[Reaction 11c]

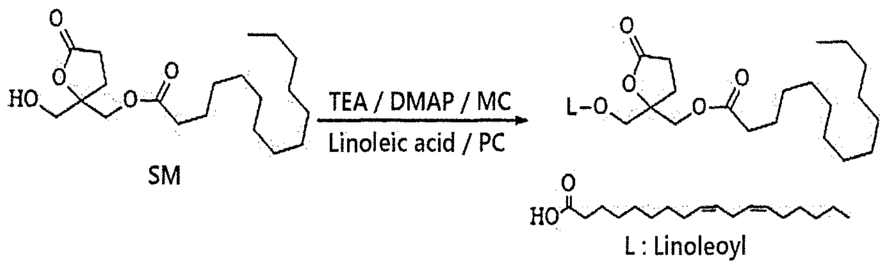


[Example 12] Synthesis of diacylglycerol lactone compound(EC-A123)

A. As shown in Reaction 12a below, 257.84mg (0.825mmole) of arachidonic acid and 43mL(0.825mmole) of pivaloyl chloride were added in 2ml of methylene chloride (MC). Then, it was cooled to 0 °C. 0.27ml(3mmole) of triethylamine(TEA) was slowly added dropwise. The reaction solution was stirred at the same temperature for 30 minutes. 150mg(0.635mmole) of the product(SM) of Reaction 1f and 7.8mg (0.0635mmole) of 4-dimethylaminopyridine(DMAP) were added at the same temperature and stirred at room temperature for 2 hours. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4 (volume ratio) mixture) to obtain 217.6mg of the target compound(yield: 64.6%).

[Reaction 12a]

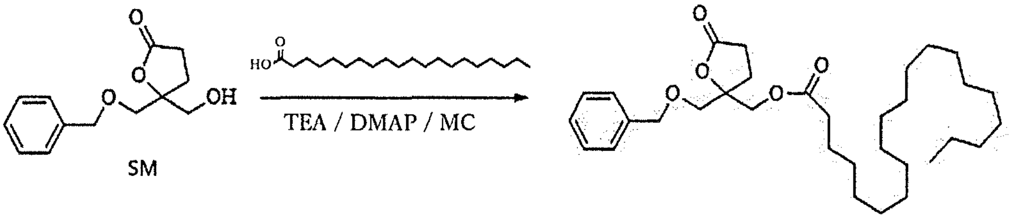


B. As shown in Reaction 12b below, 217.6mg(0.498mmole) of the product(SM) of Reaction 12a was dissolved in 2ml of methylene chloride(MC). Then, after bubbling with nitrogen gas($N_2$), it was cooled to -78 °C. 1.23ml(1.23mmole) of boron trichloride(1M in MC, $BCl_3$) was slowly added dropwise. The reaction solution was stirred at the same temperature for 2 hours. The reaction was confirmed by TLC(EA:Hex=1:2). When the reaction was completed, extraction was carried out with 0.1N-hydrochloric acid aqueous solution(c-HCl soln.)/methylene chloride(MC) and then concentrated. It was obtained 153mg of the target compound(yield: 84.7%).

[Reaction 12b]

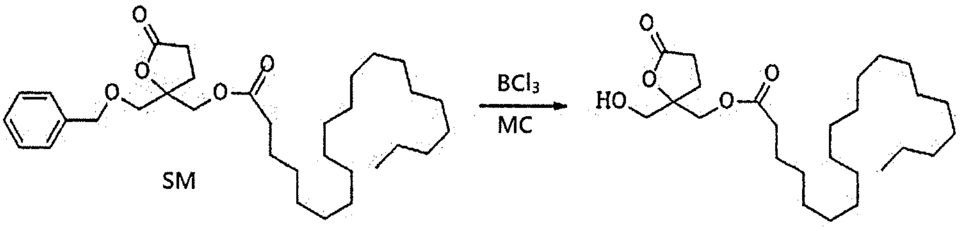


C. As shown in Reaction 12c below, 99.3 mg(0.3542mmole) of linoleic acid and 42.7mL(0.3472mmole) of pivaloyl chloride were added in 1ml of methylene chloride(MC). Then, it was cooled to 0 °C. 0.15ml(1.0416mmole) of triethylamine(TEA) was slowly added dropwise. The reaction solution was stirred at the same temperature for 30 minutes. 153mg (0.3472mmole) of the product (SM) of Reaction 12b and 4.2mg (0.035mmole) of 4-dimethylaminopyridine (DMAP) were added at the same temperature and stirred at room temperature for overnight. The reaction was confirmed by TLC(EA:Hex=1:2). When the reaction was completed, it was extracted with 0.1N-potassium hydroxide solution (KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:5 (volume ratio) mixture) to obtain 113.2mg of the target compound (yield: 46.3%).

[Reaction 12c]

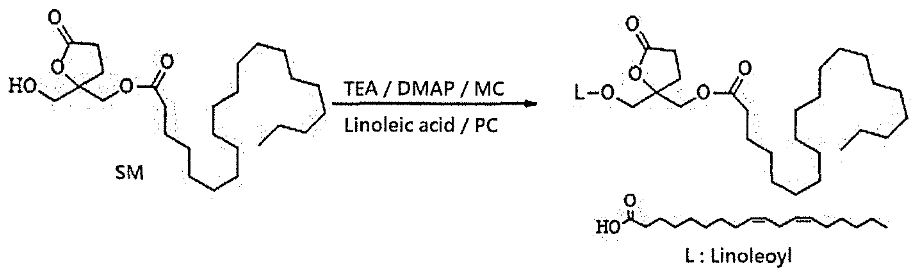


[Example 13] Synthesis of diacylglycerol lactone compound(EC-A124)

A. As shown in Reaction 13a below, 150mg (0.635mmole) of the product(SM) of Reaction 1f was dissolved in 2ml of methylene chloride(MC). Then, 0.18ml of triethylamine(TEA) and 7.8mg of 4-dimethylaminopyridine (DMAP) were added and stirred. Then, it was stirred at room temperature for 30 minutes. 87mL(0.825mmole) of isobutyryl chloride

was slowly added dropwise in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:4(volume ratio) mixture) to obtain 111.7mg of the product(yield: 57.4%).

[Reaction 13a]

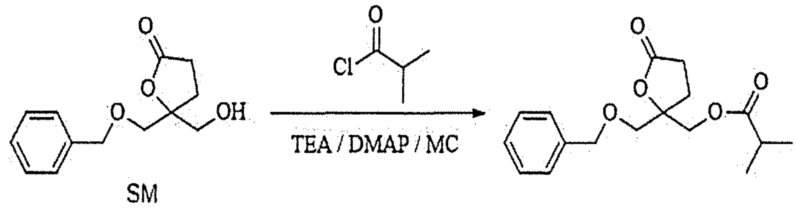


B. As shown in Reaction 13b below, 111.7mg(0.3646mmole) of the product(SM) of Reaction 13a was dissolved in 2ml of methylene chloride(MC). Then, after bubbling with nitrogen gas($N_2$), it was cooled to -78 °C. 1.1ml(1.09mmole) of boron trichloride(1M in MC, $BCl_3$) was slowly added dropwise. The reaction solution was stirred at the same temperature for 2 hours. The reaction was confirmed by TLC(EA:Hex=1:1). When the reaction was completed, extraction was carried out with 0.1N-hydrochloric acid aqueous solution(c-HCl soln.)/methylene chloride(MC) and concentrated. The concentrate was purified with a flash column(eluent: ethyl acetate(EA): hexane(Hex) = 1:1(volume ratio) mixture) to obtain 61.7mg of the product(yield: 78.3%).

[Reaction 13b]

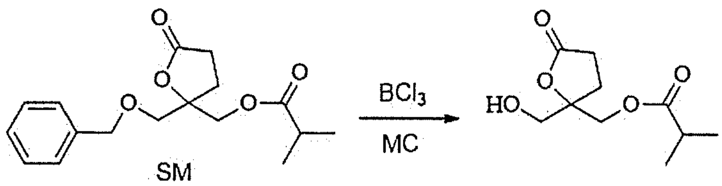


C. As shown in Reaction 13c below, 81.6mg(0.291 mmole) of linoleic acid and 37mL(0.3012mmole) of pivaloyl chloride were added in 1ml of methylene chloride(MC). Then, it was cooled to 0 °C. 0.12ml (0.8559mmole) of triethylamine(TEA) was slowly added dropwise and stirred at the same temperature for 30 minutes. 61.7mg (0.2853mmole) of the product(SM) of Reaction 13b and 3.5mg(0.029mmole) of 4-dimethylaminopyridine (DMAP) were added at the same temperature and stirred at room temperature for overnight. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:5 (volume ratio) mixture) to obtain 110.91mg of the target compound(yield: 81.2%).

[Reaction 13c]

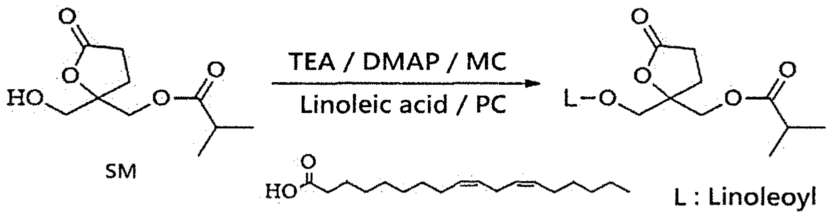


[Example 14] Synthesis of diacylglycerol lactone compound(EC-A125)

A. As shown in Reaction 14a below, 150mg (0.635mmole) of the product(SM) of Reaction 1f was dissolved in 2ml of methylene chloride(MC). Then, 0.18ml(1.27mmole) of triethylamine(TEA) and 7.8mg(0.064mmole) of 4-dimethylaminopyridine(DMAP) were added and stirred. Then, it was stirred at room temperature for 30 minutes. 0.1ml (0.825mmole) of pivaloyl chloride was slowly added dropwise and stirred at room temperature for 2 hours. The reaction was confirmed by TLC(EA:Hex=1:1.5). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:4(volume ratio) mixture) to obtain 117.5mg of the product(yield: 57.7%).

[Reaction 14a]

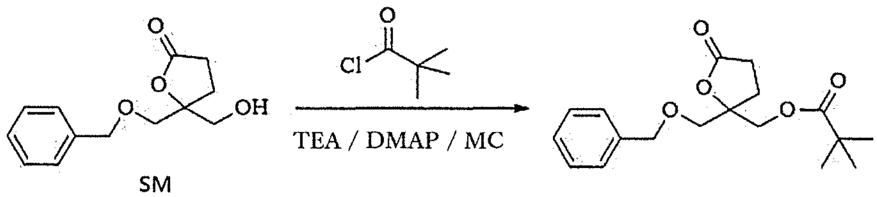


SM

B. As shown in Reaction 14b below, 117mg(0.3652mmole) of the product(SM) of Reaction 14a was dissolved in 1.83ml of methylene chloride(MC). Then, after bubbling with nitrogen gas($N_2$), it was cooled to -78 °C. 1.1ml(1.09mmole) of boron trichloride(1M in MC, $BCl_3$) was slowly added dropwise. The reaction solution was stirred at the same temperature for 2 hours. The reaction was confirmed by TLC(EA:Hex=1:2). When the reaction was completed, extraction was carried out with 0.1N-hydrochloric acid aqueous solution(c-HCl soln.)/methylene chloride(MC) and then concentrated. It was obtained 73.4mg of the target compound(yield: 87.3%).

[Reaction 14b]

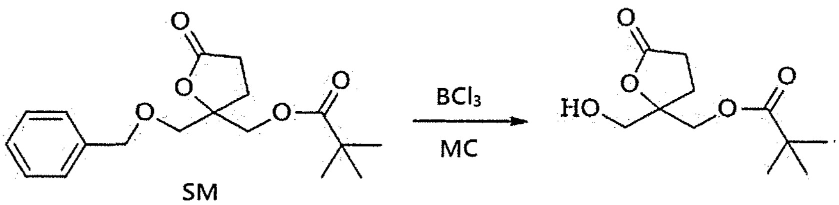


SM

C. As shown in Reaction 14c below, 91.2mg(0.3251mmole) of linoleic acid and 40mL(0.31877mmole) of pivaloyl chloride were added in 1ml of methylene chloride (MC). Then, it was cooled to 0 °C. 0.13ml(0.9563mmole) of triethylamine(TEA) was slowly added dropwise. It was stirred at the same temperature for 30 minutes. 73.4mg(0.31877mmole) of the product(SM) of Reaction 14b and 4mg(0.032mmole) of 4-dimethylaminopyridine(DMAP) were added at the same temperature and stirred at room temperature for overnight. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:5 (volume ratio) mixture) to obtain 73.6mg of the target compound(yield: 46.9%).

[Reaction 14c]

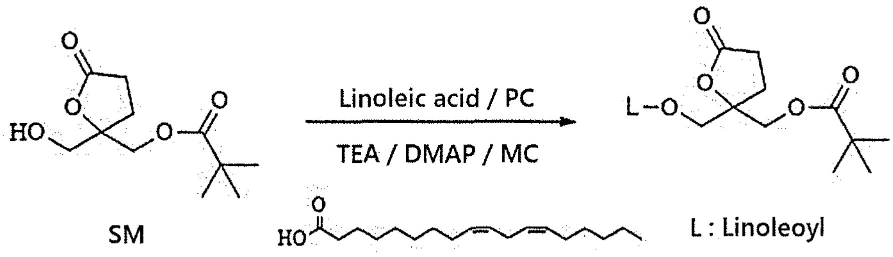


[Example 15] Synthesis of diacylglycerol lactone compound(EC-A126)

A. As shown in Reaction 15a below, 150mg(0.635mmole) of the product(SM) of Reaction 1f was dissolved in 2ml of methylene chloride(MC). Then, 0.18ml(1.27mmole) of triethylamine(TEA) and 7.8mg(0.064mmole) of 4-dimethylaminopyridine(DMAP) were added and stirred. It was stirred at 0 °C for 30 minutes. 71mL(0.825mmole) of 2-methylbutyryl chloride was added in the reaction solution and stirred at room temperature for 2 hours. The reaction was confirmed by TLC(EA:Hex=1:2). When the reaction was completed, extraction was carried out with 0.1N-hydrochloric acid aqueous solution(c-HCl soln.)/methylene chloride(MC) and then concentrated. It was obtained 102.4mg of the target compound(yield: 50.3%).

[Reaction 15a]

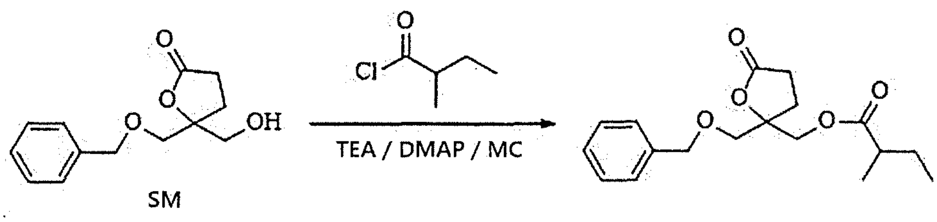


B. As shown in Reaction 15b below, 102mg(0.31842mmole) of the product(SM) of Reaction 15a was dissolved in 1.6ml of methylene chloride(MC). Then, after bubbling with nitrogen gas($N_2$), it was cooled to -78 °C. 1.0ml(0.955mmole) of boron trichloride(1M in MC, $BCl_3$) was slowly added dropwise. The reaction solution was stirred at the same temperature for 2 hours. The reaction was confirmed by TLC (EA:Hex=1:2). When the reaction was completed, extraction was carried out with 0.1N-hydrochloric acid aqueous solution(c-HCl soln.)/methylene chloride(MC) and then concentrated. It was obtained 58.5mg of the target compound(yield: 79.8%).

[Reaction 15b]

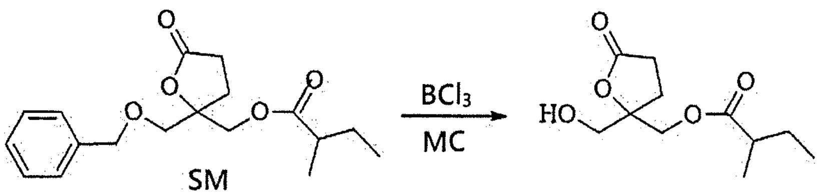


C. As shown in Reaction 15c below, 72.7mg(0.259mmole) of linoleic acid and 31mL(0.254mmole) of pivaloyl chloride were added in 1ml of methylene chloride(MC). Then, it was cooled to 0 °C. 0.11ml(0.762mmole) of triethylamine(TEA) was slowly added dropwise. It was stirred at the same temperature for 30 minutes. 58.5mg (0.254mmole) of the product(SM) of Reaction 15b and 3.1mg(0.0254mmole) of 4-dimethylaminopyridine(DMAP) were added at the same temperature and stirred at room temperature for overnight. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:5 (volume ratio) mixture) to obtain 64.7mg

of the target compound(yield: 51.7%).

[Reaction 15c]

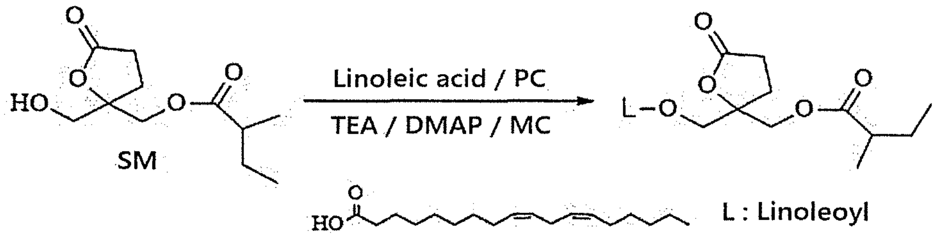


[Example 16] Synthesis of diacylglycerol lactone compound(EC-A127)

A. As shown in Reaction 16a below, 150mg(0.635mmole) of the product(SM) of Reaction 1f was dissolved in 2ml of methylene chloride(MC). Then, 0.18ml(1.27mmole) of triethylamine(TEA) and 7.8mg(0.064mmole) of 4-dimethylaminopyridine(DMAP) were added and stirred. Then, it was stirred at 0 °C for 30 minutes. 75mL(0.825mmole) of cyclopropanecarbonyl chloride was added in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:3.5(volume ratio) mixture) to obtain 123.3mg of the product(yield: 63.8%).

[Reaction 16a]

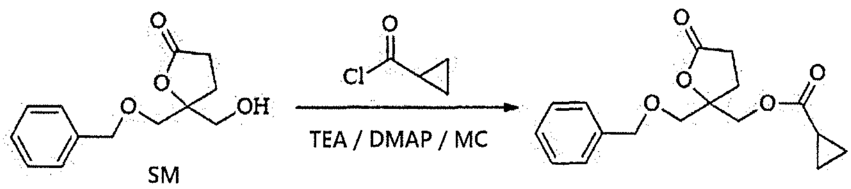


B. As shown in Reaction 15b below, 123.3mg(0.405mmole) of the product(SM) of Reaction 15a was dissolved in 1.4ml of methylene chloride(MC). Then, after bubbling with nitrogen gas($N_2$), it was cooled to -78 °C. 1.2ml(1.215mmole) of boron trichloride(1M in MC, $BCl_3$) was slowly added dropwise. The reaction solution was stirred at the same temperature for 2 hours. The reaction was confirmed by TLC (EA:Hex=1:2). When the reaction was completed, extraction was carried out with 0.1N-hydrochloric acid aqueous solution(c-HCl soln.)/methylene chloride(MC) and concentrated. The concentrate was purified with a flash column(eluent: ethyl acetate(EA): hexane(Hex) = 1:1(volume ratio) mixture) to obtain 50mg of the product(yield: 57.6%).

[Reaction 16b]

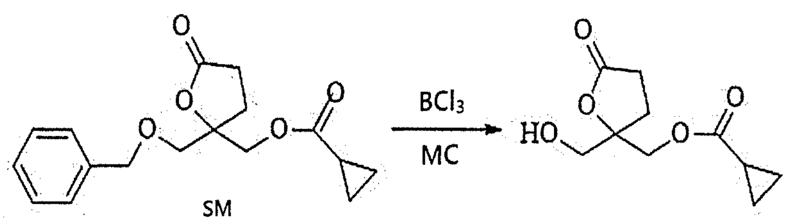


C. As shown in Reaction 16c below, 66.76mg(0.238mmole) of linoleic acid and 29mL(0.2334mmole) of pivaloyl chloride were added in 1ml of methylene chloride(MC). Then, it was cooled to 0 °C. 0.1ml(0.7002mmole) of triethylamine(TEA) was slowly added dropwise. The reaction solution was stirred at the same temperature for 30 minutes. 50mg(0.2334mmole) of the product(SM) of Reaction 16b and 2.85mg (0.023mmole) of 4-dimethylaminopyridine(DMAP) were added at the same temperature and stirred at room temperature for overnight. The reaction was

confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4 (volume ratio) mixture) to obtain 29.70mg of the target compound(yield: 62.3%).

[Reaction 16c]

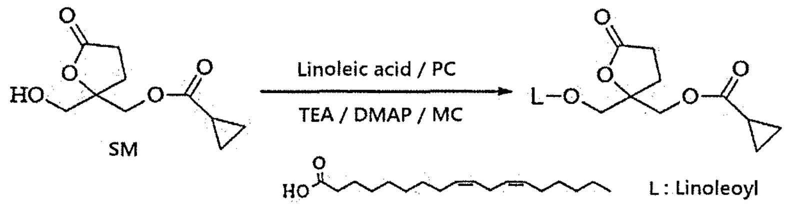


[Example 17] Synthesis of diacylglycerol lactone compound(EC-A128)

A. As shown in Reaction 17a below, 150mg(0.635mmole) of the product(SM) of Reaction 1f was dissolved in 2ml of methylene chloride(MC). Then, 0.18ml(1.27mmole) of triethylamine(TEA) and 7.8mg(0.064mmole) of 4-dimethylaminopyridine(DMAP) were added and stirred. Then, it was stirred at 0 °C for 30 minutes. 0.11ml (0.825mmole) of cyclohexanecarbonyl chloride was added in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:3.5(volume ratio) mixture) to obtain 140.9mg of the product(yield: 64%).

[Reaction 17a]

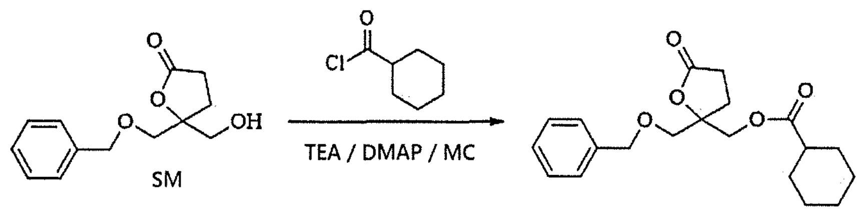


B. As shown in Reaction 17b below, 140.9mg(0.4067mmole) of the product(SM) of Reaction 17a was dissolved in 1.4ml of methylene chloride(MC). Then, after bubbling with nitrogen gas($N_2$), it was cooled to -78 °C. 1.2ml(1.22mmole) of boron trichloride(1M in MC, $BCl_3$) was slowly added dropwise. The reaction solution was stirred at the same temperature for 2 hours. The reaction was confirmed by TLC(EA:Hex=1:2). When the reaction was completed, extraction was carried out with 0.1N-hydrochloric acid aqueous solution(c-HCl soln.)/methylene chloride(MC) and then concentrated. The concentrate was purified with a flash column(eluent: ethyl acetate(EA): hexane(Hex) = 1:1(volume ratio) mixture) to obtain 95.1mg of the product(yield: 91.2%).

[Reaction 17b]

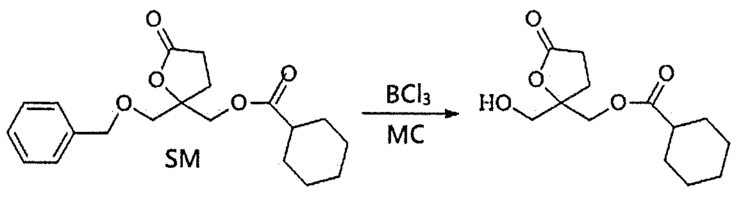


C. As shown in Reaction 17c below, 106.1 mg(0.378mmole) of linoleic acid and 45.6mL(0.371 mmole) of pivaloyl

chloride were added in 1ml of methylene chloride(MC). Then, it was cooled to 0 °C. 0.15ml(1.113mmole) of triethylamine(TEA) was slowly added dropwise. The reaction solution was stirred at the same temperature for 30 minutes. 95.1mg (0.371mmole) of the product(SM) of Reaction 17b and 4.5mg (0.037mmole) of 4-dimethylaminopyridine(DMAP) were added at the same temperature and stirred at room temperature for overnight. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4 (volume ratio) mixture) to obtain 126.4mg of the target compound(yield: 65.7%).

[Reaction 17c]

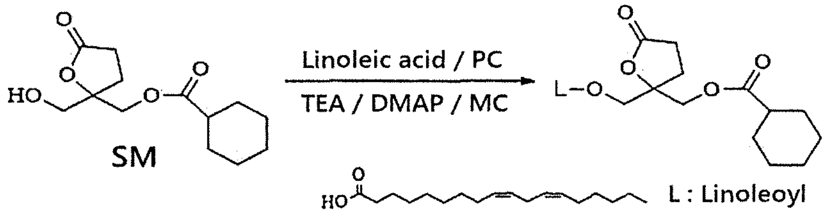


[Example 18] Synthesis of diacylglycerol lactone compound(EC-A130)

A. As shown in Reaction 18a below, 5.88g(22.945mmole) of palmitic acid and 2.88ml (22.945mmole) of pivaloyl chloride were added in 1ml of methylene chloride (MC). Then, it was cooled to 0 °C. 7.4ml (52.95mmole) of triethylamine(TEA) was slowly added dropwise. The reaction solution was stirred at the same temperature for 30 minutes. 1.47g(17.65mmole) of the product(SM) of Reaction 1f and 216mg (1.765mmole) of 4-dimethylaminopyridine(DMAP) were added at the same temperature and stirred at room temperature for overnight. The reaction was confirmed by TLC (EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4.5 (volume ratio) mixture) to obtain 1.9g of the target compound(yield: 22.6%).

[Reaction 18a]

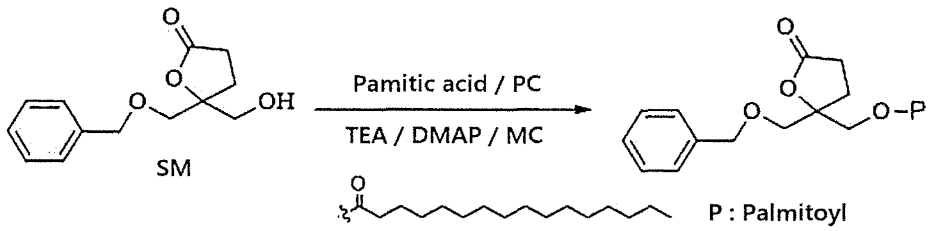


B. As shown in Reaction 18b below, 1.9g (4.003mmole) of the product(SM) of Reaction 18a was dissolved in 20ml of methylene chloride(MC). Then, after bubbling with nitrogen gas($N_2$), it was cooled to -78 °C. 12ml(12.01mmole) of boron trichloride(1M in MC, $BCl_3$) was slowly added dropwise. The reaction solution was stirred at the same temperature for 2 hours. The reaction was confirmed by TLC(EA:Hex=1:1). When the reaction was completed, extraction was carried out with 0.1N-hydrochloric acid aqueous solution(c-HCl soln.)/methylene chloride(MC) and then concentrated. The concentrate was purified with a flash column(eluent: ethyl acetate(EA): hexane(Hex) = 1:1.5 (volume ratio) mixture) to obtain 1.287g of the target compound(yield: 83.6%).

[Reaction 18b]

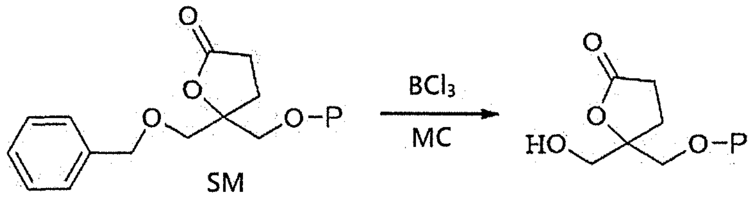


C. As shown in Reaction 18c below, 100mg(0.26mmole) of the product(SM) of Reaction 18b was dissolved in 1ml of methylene chloride(MC). Then, 72.5mL (0.52mmole) of triethylamine(TEA) and 3.17mg(0.026mmole) of 4-dimethylaminopyridine (DMAP) were added and stirred. Then it was stirred at 0 °C for 30 minutes. 35.3mL (0.338mmole) of butyryl chloride was added in the reaction solution. it was stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column (eluent: ethyl acetate(EA): hexane(Hex) = 1:3(volume ratio) mixture) to obtain 86.8mg of the target compound (yield: 73.4%).

[Reaction 18c]

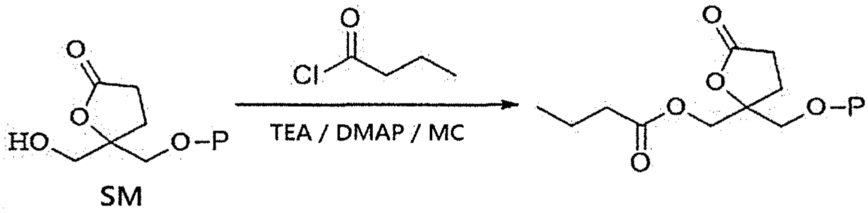


[Example 19] Synthesis of diacylglycerol lactone compound(EC-A131)

As shown in Reaction 19a below, 100mg(0.26mmole) of the product(SM) of Reaction 18b was dissolved in 1ml of methylene chloride(MC). Then, 72.5mL (0.52mmole) of triethylamine (TEA) and 3.17mg(0.026mmole) of 4-dimethylaminopyridine(DMAP) were added and stirred. Then, it was stirred at 0 °C for 30 minutes. 36mL (0.338mmole) of isobutyryl chloride was added in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:4(volume ratio) mixture) to obtain 86.8mg of the product(yield: 73.4%).

[Reaction 19a]

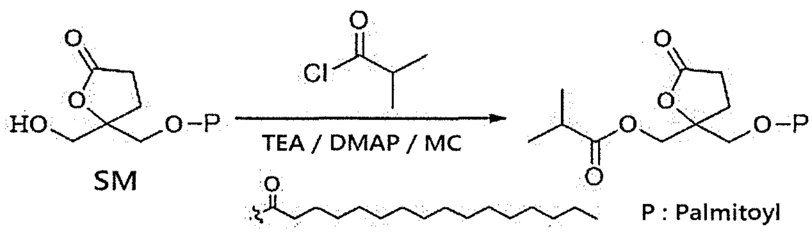


[Example 20] Synthesis of diacylglycerol lactone compound(EC-A132)

As shown in Reaction 20a below, 100mg(0.26mmole) of the product(SM) of Reaction 18b was dissolved in 1ml of methylene chloride(MC). Then, 72.5mL (0.52mmole) of triethylamine(TEA) and 3.17mg(0.026mmole) of 4-dimethylaminopyridine (DMAP) were added and stirred. Then, it was stirred at 0 °C for 30 minutes. 41.6mL (0.338mmole) of pivaloyl chloride was added in the reaction solution and stirred at room temperature for 2 hours. The reaction was

confirmed by TLC (EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:4(volume ratio) mixture) to obtain 84.9mg of the product(yield: 69.7%).

[Reaction 20a]

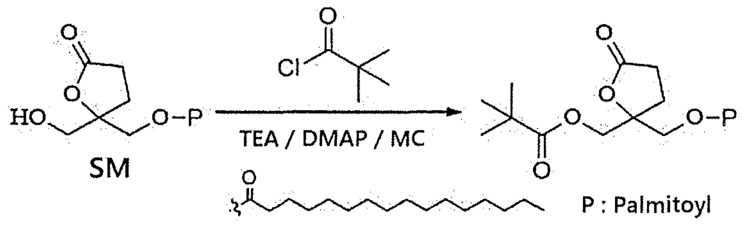


[Example 21] <u>Synthesis of diacylglycerol lactone compound(EC-A133)</u>

As shown in Reaction 21a below, 100mg (0.26mmole) of the product(SM) of Reaction 18b was dissolved in 1ml of methylene chloride(MC). Then, 0.1ml(0.78mmole) of triethylamine(TEA) and 3.17mg(0.026mmole) of 4-dimethylaminopyridine(DMAP) were added and stirred. Then, it was stirred at 0 °C for 30 minutes. 29mL (0.338mmole) of 2-methylbutyryl chloride was added in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:4(volume ratio) mixture) to obtain 87.6mg of the product(yield: 71.9%).

[Reaction 21a]

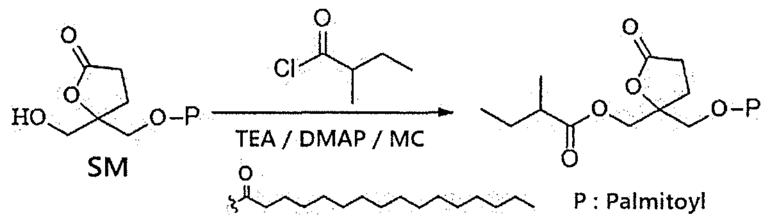


[Example 22] <u>Synthesis of diacylglycerol lactone compound(EC-A134)</u>

As shown in Reaction 22a below, 100mg (0.26mmole) of the product(SM) of Reaction 18b was dissolved in 1ml of methylene chloride(MC). Then, 72.5mL (0.52mmole) of triethylamine(TEA) and 3.17mg(0.026mmole) of 4-dimethylaminopyridine (DMAP) were added and stirred. Then, it was stirred at 0 °C for 30 minutes. 30.7mL(0.338mmole) of cyclopropanecarbonyl chloride was added in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate (EA): hexane(Hex) = 1:4(volume ratio) mixture) to obtain 89.3mg of the product (yield: 75.8%).

[Reaction 22a]

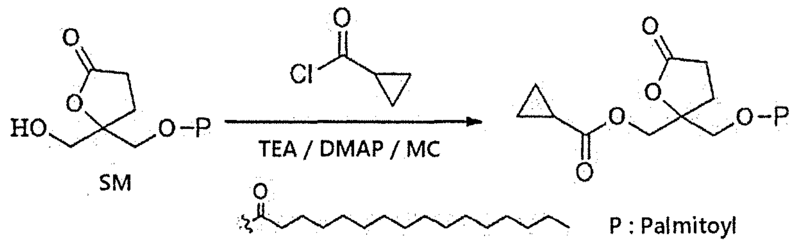

[Example 23] Synthesis of diacylglycerol lactone compound(EC-A135)

As shown in Reaction 23a below, 100mg(0.26mmole) of the product(SM) of Reaction 18b was dissolved in 1ml of methylene chloride(MC). Then, 72.5mL(0.52mmole) of triethylamine(TEA) and 3.17mg(0.026mmole) of 4-dimethylaminopyridine(DMAP) were added and stirred. Then, it was stirred at 0 °C for 30 minutes. 45.2mL (0.338mmole) of cyclopropanecarbonyl chloride was added in the reaction solution and stirred at room temperature for 2 hours. The reaction was confirmed by TLC(EA:Hex=1:2). When the reaction was completed, it was extracted with 0.1N-hydrochloric acid solution(c-HCl soln.)/methylene chloride(MC) and then concentrated. The concentrate was purified with a flash column(eluent: ethyl acetate(EA): hexane(Hex) = 1:4(volume ratio) mixture) to obtain 88.1mg of the product (yield: 68.5%).

[Reaction 23a]

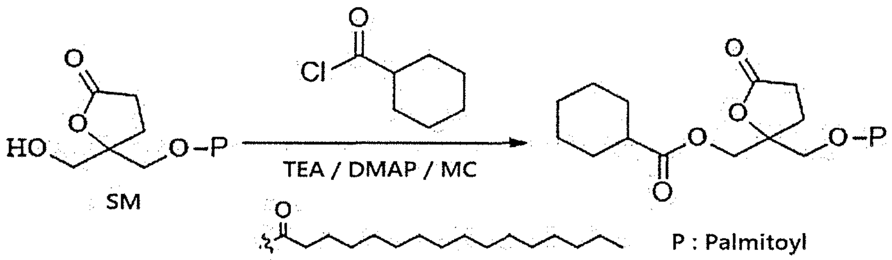


[Example 24] Synthesis of diacylglycerol lactone compound(EC-A136)

As shown in Reaction 24a below, 67.7mg(0.338mmole) of lauric acid and 42mL (0.338mmole) of pivaloyl chloride were added in 1ml of methylene chloride(MC). Then, it was cooled at 0 °C. 0.1ml (0.78mmole) of triethylamine(TEA) was slowly added dropwise at the same temperature. It was stirred at the same temperature for 30 minutes. 100mg (0.26mmole) of the product(SM) of Reaction 18b and 3.17mg (0.026mmole) of 4-dimethylaminopyridine(DMAP) were added at the same temperature. It was stirred at room temperature for overnight. The reaction was confirmed by TLC(EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4 (volume ratio) mixture) to obtain 103.6mg of the target compound(yield: 70.3%).

[Reaction 24a]

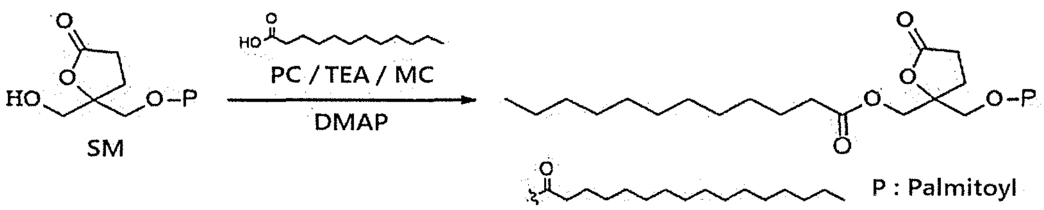


[Example 25] Synthesis of diacylglycerol lactone compound(EC-A137)

As shown in Reaction 25a below, 77.2mg (0.338mmole) of myristic acid and 42mL (0.338mmole) of pivaloyl chloride were added in 1ml of methylene chloride(MC). Then, it was cooled at 0 °C. 0.1ml(0.78mmole) of triethylamine(TEA) was slowly added dropwise at the same temperature. It was stirred at the same temperature for 30 minutes. 100mg(0.26mmole) of the product(SM) of Reaction 18b and 3.17mg (0.026mmole) of 4-dimethylaminopyridine(DMAP) were added at the same temperature. It was stirred at room temperature for overnight. The reaction was confirmed by TLC (EA:Hex=1:2). When the reaction was completed, it was extracted with 0.1N-potassium hydroxide solution(KOH soln.)/methylene chloride(MC). The extract solution was extracted with 0.07N-hydrochloric acid solution(c-HCl soln.)/methylene chloride(MC) and then concentrated after removing moisture with magnesium sulfate($MgSO_4$). The concentrate was purified with a flash column(eluent: ethyl acetate(EA): hexane(Hex) = 1:4 (volume ratio) mixture) to obtain 106.4mg of the target compound(yield: 68.8%).

[Reaction 25a]

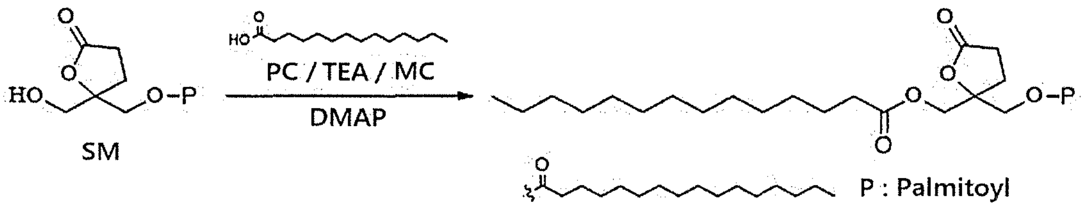


[Experimental Example 1-1] Cytotoxicity assessment of diacylglycerol lactone compound

In DMEM (Dulbecco Modified Eagle Medium) medium with 10% Fetal Bovine Serum added, RAW264.7 cells, which are cells of the mouse macrophage family, were suspended at a concentration of 1 x $10^5$ cells/ml, and then were inoculated into 96 well plates by 100 μl, and culture was conducted for 15 hours. Next, the culture solution was treated with a diacylglycerol lactone compound having the kind and concentration as shown in Table 1 below, and then the additional culture was carried out for 24 hours. According to the EZ-CYTOX (Daeillab_EZ-1000) manual for measuring the amount of living cells using WST, 10 μl of EZ-Cytox was added to each well and reacted for up to 2 hours from 30 minutes, and then an optical density (OD) at 450 nm was measured. Cell viability was calculated according to Equation 1 below, and the results thereof are shown together in Table 1.

[Equation 1]

$$Cell\ viability\ (\%) = \left(\frac{OD\ \ at\ 450nm\ of\ Treatment\ group\ of\ diacylglycerol\ lactone\ compound}{OD\ at\ 450nm\ of\ negative\ control\ goup}\right)X\ 100$$

[Table 1]

| | sample | concentration(μg/mℓ) | RAW267.4 cell viability (%, average ± deviation) |
|---|---|---|---|
| 1 | Negative control group | 0 | 107.09 ± 10.03 |
| 2 | EC-A115 | 100 | 145.03 ± 11.86 |
| 3 | EC-A116 | 100 | 141.09 ± 5.01 |
| 4 | EC-A117 | 100 | 111.93 ± 0.09 |
| 5 | EC-A118 | 100 | 126.45 ± 10.58 |
| 6 | EC-A119 | 100 | 116.45 ± 13.41 |
| 7 | EC-A120 | 100 | 106.83 ± 17.7 |
| 8 | EC-A121 | 100 | 114.25 ± 33.11 |
| 9 | EC-A122 | 100 | 105.48 ± 3.92 |
| 10 | EC-A123 | 100 | 107.35 ± 3.46 |
| 11 | EC-A124 | 100 | 146.77 ± 647 |
| 12 | EC-A125 | 100 | 106 ± 9.94 |
| 13 | EC-A126 | 100 | 107.74 ± 0.91 |
| 14 | EC-A127 | 100 | 110.25 ± 4.47 |
| 15 | EC-A128 | 100 | 129.35 ± 21.98 |
| 16 | EC-A129 | 100 | 103.93 ± 0.27 |
| 17 | EC-A130 | 100 | 118.51 ± 34.21 |

(continued)

| | sample | concentration($\mu$g/m$\ell$) | RAW267.4 cell viability (%, average ± deviation) |
|---|---|---|---|
| 18 | EC-A131 | 100 | 100.25 ± 14.78 |
| 19 | EC-A132 | 100 | 96.58 ± 5.01 |
| 20 | EC-A133 | 100 | 114.45 ± 3.46 |
| 21 | EC-A134 | 100 | 110.06 ± 4.19 |
| 22 | EC-A135 | 100 | 104 ± 3.28 |
| 23 | EC-A136 | 100 | 95.54 ± 3.01 |
| 24 | EC-A137 | 100 | 93.87 ± 8.85 |

As shown in Table 1, from the results of observing the cell viability of RAW264.7 cells dependent on the diacylglycerol lactone compound of the present invention, it was confirmed that all compounds did not exhibit cytotoxicity at a concentration of 100 μg/ml.

[Experimental Example 2-1] Increase in expression of CXCL8 (IL-8) of diacylglycerol lactone compound

In RPMI (Hyclone, Thermo Scientific) medium to which 10% Fetal Bovine Serum was added, THP-1 cells, a human macrophage family, were suspended at a concentration of 1 x $10^5$ cells/ml, and culture was conducted in a 5% $CO_2$ humidified incubator at 37 °C. The cultured THP-1 cells were inoculated into a 12 well plate by 1 x 106 cells/ml and stabilized for 30 minutes. Then, the culture solution was treated with a diacylglycerol lactone compound of the type shown in Table 2 below for 1 hour and then was treated with Gemcitabine (2 μg/ml) of a cell stimulator, and subsequent further incubation was conducted for 24 hours. Thereafter 1.5 ml of the culture supernatant was collected for each well and centrifuged (at 3000 rpm, 5 minutes) to recover the supernatant. The CXCL8 (IL-8) level in the recovered supernatant was measured according to the manual provided by the human IL-8 ELISA set (BD Biosciences). The day before ELISA was carried out, the IL-8 capture antibody was diluted in phosphate buffered saline, coated on a microwell, and then stored at 4 °C overnight. Each well was washed three times with a washing buffer solution and then blocked with 2% Bovine Serum Albumin (BSA) for 1 hour at room temperature. After washing with washing buffer solution three times, 100μl of sample was dispensed into each well and left at room temperature for 2 hours. Detection antibody which was washed 3 times with washing buffer and diluted was dispensed into each well and allowed to react at room temperature for 1 hour and left at room temperature for 1 hour. Thereafter, the secondary HRP conjugated antibody was reacted at room temperature for 30 minutes, washed three times with a washing buffer, and treated with 50 μl of stop solution for each well, and then the optical density was measured at 450 nm with an ELISA microplate leader. The results of the expression increase rate are shown in Table 2 and FIG. 1 below.

[Table 2]

| | sample | Concentration ($\mu$g/m$\ell$) | CXCL8 [IL-8] concentration (pg/$\mu\ell$, average ± deviation) |
|---|---|---|---|
| 1 | Negative control group | 0 | 20.54 ± 2.50 |
| 2 | Gemcitabine | 2 | 101.83 ± 1.59 |
| 3 | EC-A115 | 100 | 1202.80 ± 38.09 |
| 4 | EC-A116 | 100 | 865.54 ± 12.31 |
| 5 | EC-A117 | 100 | 568.12 ± 8.21 |
| 6 | EC-A118 | 100 | 622.16 ± 661 |
| 7 | EC-A119 | 100 | 343.61 ± 7.75 |
| 8 | EC-A120 | 100 | 237.80 ± 3.64 |
| 9 | EC-A121 | 100 | 215.38 ± 4.33 |
| 10 | EC-A122 | 100 | 190.06 ± 4.56 |
| 11 | EC-A123 | 100 | 144.25 ± 17.33 |

(continued)

| | sample | Concentration ($\mu$g/m$\ell$) | CXCL8 [IL-8] concentration (pg/$\mu\ell$, average $\pm$ deviation) |
|---|---|---|---|
| 12 | EC-A124 | 100 | 663.45 $\pm$ 2.96 |
| 13 | EC-A125 | 100 | 252 $\pm$ 11.86 |
| 14 | EC-A126 | 100 | 352.96 $\pm$ 0.45 |
| 15 | EC-A127 | 100 | 532.32 $\pm$ 1.82 |
| 16 | EC-A128 | 100 | 351.03 $\pm$ 7.75 |
| 17 | EC-A129 | 100 | 1145.87 $\pm$ 15.5 |
| 18 | EC-A130 | 100 | 1239.25 $\pm$ 39.91 |
| 19 | EC-A131 | 100 | 1256.19 $\pm$ 15.96 |
| 20 | EC-A132 | 100 | 921.35 $\pm$ 52.46 |
| 21 | EC-A133 | 100 | 1176.67 $\pm$ 29.42 |
| 22 | EC-A134 | 100 | 1266.03 $\pm$ 2.05 |
| 23 | EC-A135 | 100 | 310.87 $\pm$ 14.37 |
| 24 | EC-A136 | 100 | 138.93 $\pm$ 2.96 |
| 25 | EC-A137 | 100 | 133.12 $\pm$ 8.43 |

From Table 2 and FIG. 1, it was confirmed that when THP-1 cells are treated with Gemcitabine, an anticancer drug, the secretion of CXCL8 (IL-8) chemokine, a neutrophil cell recruitment factor, is increased by about five times compared to the negative control group, and addition of diacylglycerol lactone compound increase CXCL8 chemokine secretion from THP-1 cells by at least 1.3 times and up to 12 times with respect to that of the anticancer treatment group.

[Experimental Example 3-1] Animal model of lung infection with bacteria and sample administration

For getting mice model whose lung are infected with bacteria, 12-week old Balb/c male mice were purchased from Koatech Corporation (South Korea) and maintained in certain pathogen-free facilities under moderate temperature and lights cycles. For obtain bacteria to induce lung infection, Aeruginosa K (PAK) of the genus Psuedomonas was incubated in LB broth or LB agar plate overnight at 37 °C, and then the culture solution was centrifuged at 13,000 x g for 2 minutes to obtain a bacterial pellet. Thereafter, the bacterial pellet was suspended in phosphate buffered saline (PBS), and the optical density of the serial dilution was measured and plated on an agar plate, so that bacterial inoculum having a colony forming unit (CFU) was obtained. For use in the following experiments, a bacterial inoculum solution for infection was prepared at a concentration of 1 x $10^5$ CFU per 20 $\mu$l.

[Experimental Example 4-1] Confirmation of CFU level in mice infected with P. aeruginosa

The PAK bacteria inoculum prepared in Experimental Example 3 (1 x $10^5$ CFU per mouse in 20 $\mu$l PBS) was administered to a total of eight 12-week-old Balb/c mice by nasal injection, wherein diacylglycerol lactone compound (EC_A129) was orally administered by 250 mg/kg to four mice among the PAK-treated groups, and PBS was administered to the control group. After 4 hours, samples of bronchoalveolar lavage fluid (BALF) were collected from the PAK-treated group. The collected BALF sample was diluted 1:1000 with PBS, and the diluted sample was plated on LB agar, and then incubated overnight at 37 °C. CFU levels in BALF were confirmed by measuring the number of surviving bacteria by a plate count method, and the results thereof are shown in Table 3, FIGS. 2 and 3 below.

[Table 3]

| Mouse subject number | Negative control group ($10^3$ CFU/m$\ell$) | PAK infected group ($10^3$ CFU/m$\ell$) | PAK + diacylglycerol lactone compound EC-A129 treated group ($10^3$ CFU/m$\ell$) |
|---|---|---|---|
| #1 | 0 | 112.0 | 59.0 |
| #2 | 0 | 246.0 | 3.0 |

(continued)

| Mouse subject number | Negative control group ($10^3$ CFU/mℓ) | PAK infected group ($10^3$ CFU/mℓ) | PAK + diacylglycerol lactone compound EC-A129 treated group ($10^3$ CFU/mℓ) |
|---|---|---|---|
| #3 | 0 | 220.0 | 16.0 |
| #4 | 0 | 60.0 | 38.0 |
| average± deviation | 0 | 160.0 ± 88.0 | 29.0 ± 25.0 |

As shown in Table 3 and FIG. 2, it was confirmed that the bacterial CFU in the BALF was rapidly increased at 4 hours after PAK administration. On the other hand, when the A129 compound which significantly increased the CXCL8 expression, a neutrophil recruiting factor, among the diacylglycerol lactone compounds, was administered together with PAK, the bacterial CFU in the alveolar lavage fluid at 4 hours was significantly lower than in the PAK alone group. As described in Experimental Example 3, BALF collected for each mouse for each group was diluted 1:1000 with PBS and applied 100 μl to an LB plate to incubate the bacteria for 16 hours and the number of bacteria then was measured. Before measuring the number of bacteria, the LB plates were photographed, which is shown in FIG. 3, illustrating that in PAK-infected mice, diacylglycerol lactone compound promotes bacterial removal in the early stages of infection.

## Claims

1. A diacylglycerol lactone compound represented by following Chemical formula 1,

[Chemical formula 1]

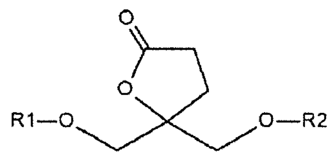

in Chemical formula 1, R1 and R2 are independently a fatty acid residue of 2 to 30 carbon atoms.

2. The diacylglycerol lactone compound of claim 1, wherein R1 and R2 are independently selected from the group consisting of acetyl, propionyl, butyryl, isobutyryl, valeroyl, pivaloyl, 2-methylbutyryl, cyclopropanecarbonyl, cyclohexanecarbonyl, hexanoyl, heptanoyl, nonanoyl, dodecanoyl, myristoyl, palmitoyl, linoleoyl, oleoyl, linolenoyl, eicosanoyl and arachidonoyl.

3. The diacylglycerol lactone compound of claim 1, wherein the diacylglycerol lactone compound of Chemical formula 1 is a compound represented by following Chemical formula 2a or Chemical formula 2b.

[Chemical formula 2a]

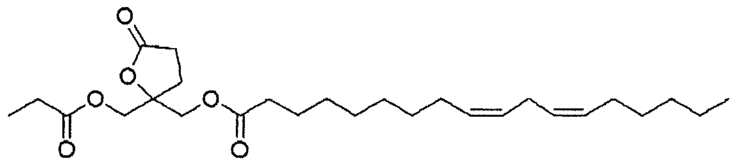

[Chemical formula 2b]

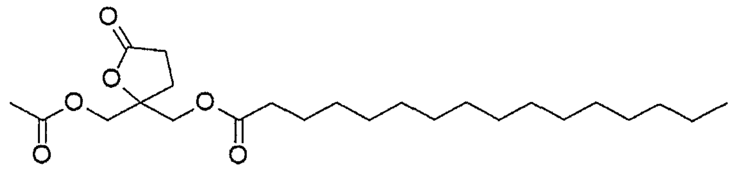

**4.** A method for preparing diacylglycerol lactone comprising the steps of:

reacting a compound represented by Chemical formula A with $CH_2$=CH-$CH_2$-MgX (X is a halogen atom) to prepare a compound represented by Chemical formula B, as shown in Reaction 1 below,

[Reaction 1]

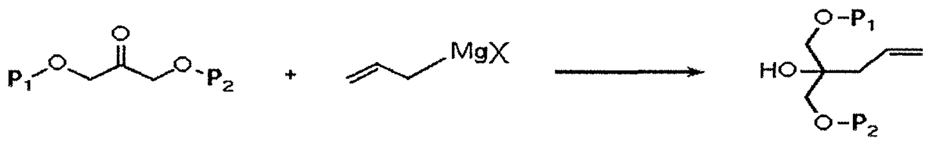

[A] [B]

in Reaction 1, P1 and P2 are protecting groups;
carrying out hydroxylation of a double bond of the compound of Chemical formula B to obtain a compound represented by Chemical formula C, as shown in Reaction 2 below,

[Reaction 2]

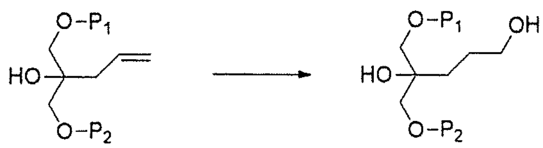

[B] [C]

in Reaction 2, P1 and P2 are protecting groups;
carrying out lacton-cyclization reaction of the compound of Chemical formula C to obtain a compound represented by Chemical formula D, as shown in Reaction 3 below,

[Reaction 3]

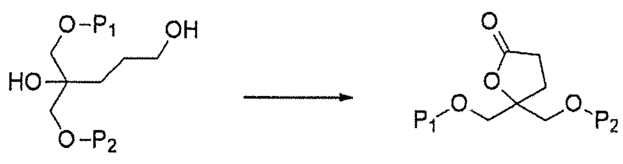

[C] [D]

in Reaction 3, P1 and P2 are protecting groups; and
carrying out deprotection reaction and esterification reaction of the compound of Chemical formula D to obtain a compound represented by Chemical formula 1, as shown in Reaction 4 below,

[Reaction 4]

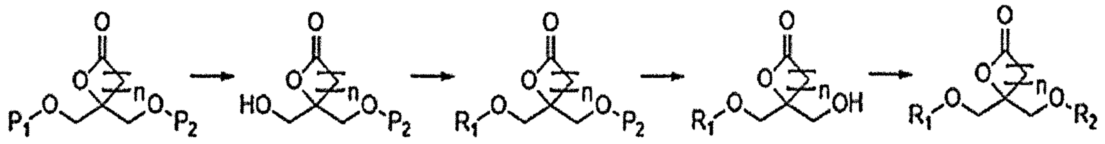

[D] [E] [F] [G] [CHEMICAL FORMULA 1]

in Reaction 4, P1 and P2 are protecting groups, and R1 and R2 are independently a fatty acid residue of 2 to 30 carbon atoms.

**5.** An immunostimulator comprising diacylglycerol lactone compound represented by following Chemical formula 1 as an active ingredient,

[Chemical formula 1]

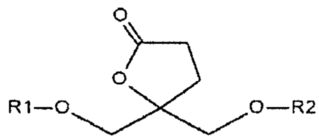

in Chemical formula 1, R1 and R2 are independently a fatty acid residue of 2 to 30 carbon atoms.

**6.** The immunostimulator of claim 5, wherein the diacylglycerol lactone compound increases IL-8 cytokine expression.

**7.** The immunostimulator of claim 5, wherein the diacylglycerol lactone compound promotes neutrophil migration from blood to cells.

**8.** The immunostimulator of claim 5, wherein the diacylglycerol lactone compound prevents or treats immune disease selected from the group consisting of bacterial or viral infections, acute and chronic inflammatory lung diseases, pneumonia and sepsis.

**9.** The immunostimulator of claim 5, wherein amount of the diacylglycerol lactone compound is 0.0001 to 100.0% by weight.

**10.** A health functional food composition for immunity enhancement, comprising diacylglycerol lactone compound represented by following Chemical formula 1 as an active ingredient,

[Chemical formula 1]

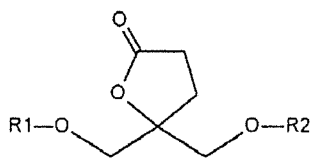

in Chemical formula 1, R1 and R2 are independently a fatty acid residue of 2 to 30 carbon atoms.

**11.** A method for immunity enhancement comprising administering to a non-human subject the immunostimulator according to any one of claims 5 to 9.

Fig. 1

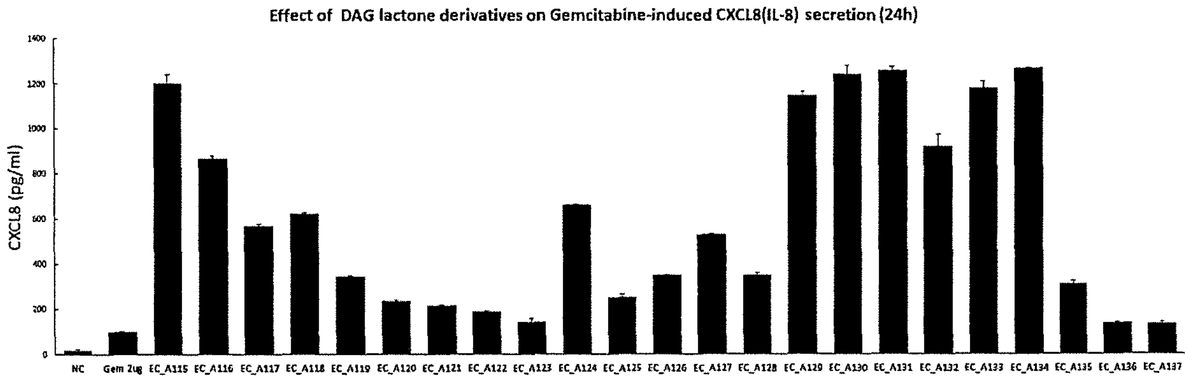


Fig. 2

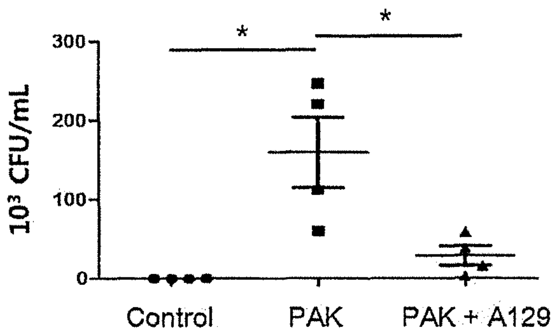


Fig. 3

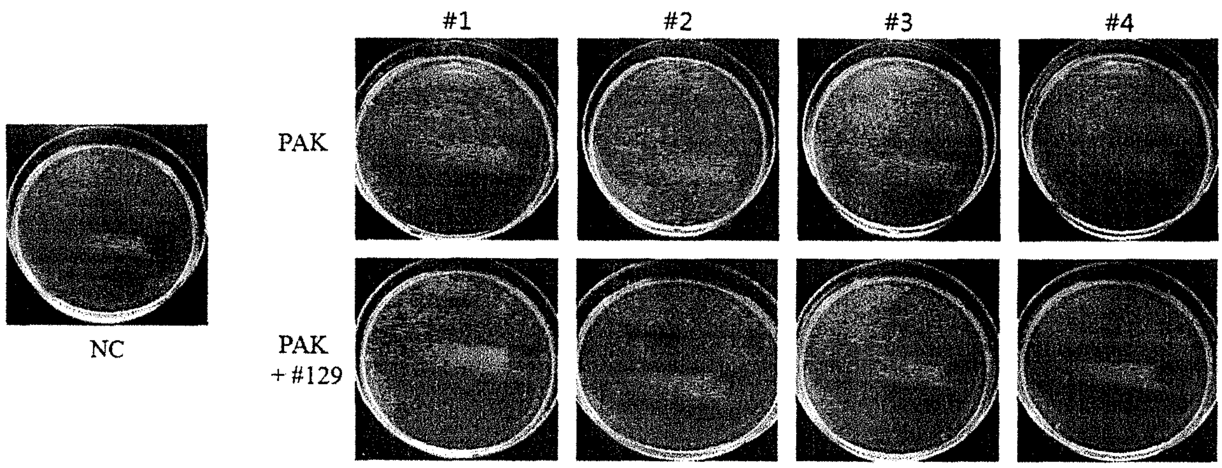

# INTERNATIONAL SEARCH REPORT

International application No.
**PCT/KR2019/002757**

A. CLASSIFICATION OF SUBJECT MATTER

***C07D 307/32(2006.01)i, A61K 31/365(2006.01)i, A23L 33/10(2016.01)i, A61P 31/00(2006.01)i, A61P 37/00(2006.01)i***

According to International Patent Classification (IPC) or to both national classification and IPC

B. FIELDS SEARCHED

Minimum documentation searched (classification system followed by classification symbols)
C07D 307/32; A61K 31/365; A23L 33/10; A61P 31/00; A61P 37/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), STN (Registry, Caplus, Marpat, Casreact) & Keywords: diacylglycerollactone, immune, virus infection, bacterium infection, lung disease

C. DOCUMENTS CONSIDERED TO BE RELEVANT

| Category* | Citation of document, with indication, where appropriate, of the relevant passages | Relevant to claim No. |
|---|---|---|
| X | CHENEVERT, R. et al. Enzymatic desymmetrization of 5-bis (hydroxymethyl) tetrahydro-2-furanone: a template for protein kinase C ligands. Tetrahedron: Asymmetry. 2004, vol. 15, pages 863-866 See abstract; page 863; and formulas 1, 2. | 1,2 |
| Y | | 3-11 |
| Y | SINGH, B. K. et al. The immunomodulatory functions of diacylglycerol kinase ζ. Frontiers in Cell and Developmental Biology. 2016, vol. 4, no. 96, pages 1-9 See abstract; and pages 1, 4. | 3,5-11 |
| Y | TAMAMURA, H. et al. Conformationally constrained analogues of diacylglycerol (DAG). 23. Hydrophobic ligand-protein interactions versus ligand-lipid interactions of DAG-lactones with protein kinase C(PK-C). Journal of Medicinal Chemistry. 2004, vol. 47, pages 4858-4864 See abstract; and formula 1. | 4 |
| Y | BAUMANN, D. O. et al. Synthesis and biological evaluation of several bryostatin analogues bearing a diacylglycerol lactone C-ring. The Journal of Organic Chemistry. 2016, vol. 81, pages 7862-7883 See abstract; and formula 3. | 4 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 JULY 2019 (02.07.2019) | **02 JULY 2019 (02.07.2019)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**

International application No.
**PCT/KR2019/002757**

C (Continuation). DOCUMENTS CONSIDERED TO BE RELEVANT

| Category* | Citation of document, with indication, where appropriate, of the relevant passages | Relevant to claim No. |
|---|---|---|
| A | MUKAIDA, N. et al. Pathophysiological roles of interleukin-8/CXCL8 in pulmonary diseases. American Journal of Physiology-Lung Cellular and Molecular Physiology. 2003, vol. 284, pages L566-L577<br>See the entire document. | 1-11 |

Form PCT/ISA/210 (continuation of second sheet) (January 2015)

**INTERNATIONAL SEARCH REPORT**
Information on patent family members

International application No.
**PCT/KR2019/002757**

| Patent document cited in search report | Publication date | Patent family member | Publication date |
|---|---|---|---|
| None | | | |

Form PCT/ISA/210 (patent family annex) (January 2015)